# EUROPEAN PATENT SPECIFICATION

(11) **EP 3 672 677 B1**
(45) Date of publication and mention of the grant of the patent: **11.11.2026**
(21) Application number: 18772898.5
(22) Date of filing: 24.08.2018
(51) Int. Cl.: A61M 27/00

(54) **INDWELLING PUMP FOR FACILITATING REMOVAL OF URINE FROM THE URINARY TRACT**
VERWEILPUMPE ZUR ERLEICHTERUNG DER ENTFERNUNG VON URIN AUS DEM HARNTRAKT
POMPE À DEMEURE POUR FACILITER L'ÉLIMINATION DE L'URINE DU TRACTUS URINAIRE

(30) Priority: 25.08.2017 US 201762550259 P
(43) Date of publication of application: 01.07.2020
(73) Proprietor: Roivios Limited, 13007 Nassau, N.P. (BS)
(72) Inventor: ORR, David, E., Piedmont, South Carolina 29673 (US); UPPERCO, Jacob, L., Atlanta, Georgia 30308 (US); ERBEY II, John, R., Milton, Georgia 30004 (US)
(74) Representative: Rentsch Partner AG
(86) International application number: PCT/IB2018/056444
(87) International publication number: WO 2019/038730

(56) References cited:
- WO-A1-2018/200050
- WO-A1-95/29716
- WO-A2-2017/015345
- WO-A2-2017/015351
- CN-A- 106 693 092
- US-A1- 2015 157 836
- US-A1- 2017 128 654
- US-B1- 9 339 636

## Description

### BACKGROUND

### Technical Field

The present disclosure relates to a pump for deployment within a body lumen and, for example, to a pump sized for insertion and deployment within a patient's urinary tract for inducing negative and/or positive pressure in a patient's bladder, ureter(s), and/or kidney(s).

### Background

The renal or urinary system includes a pair of kidneys, each kidney being connected by a ureter to the bladder, and a urethra for draining urine produced by the kidneys from the bladder. The kidneys perform several vital functions for the human body including, for example, filtering the blood to eliminate waste in the form of urine. The kidneys also regulate electrolytes (e.g., sodium, potassium and calcium) and metabolites, blood volume, blood pressure, blood pH, fluid volume, production of red blood cells, and bone metabolism. Adequate understanding of the anatomy and physiology of the kidneys is useful for understanding the impact that altered hemodynamics and other fluid overload conditions have on their function.

In normal anatomy, the two kidneys are located retroperitoneally in the abdominal cavity. The kidneys are bean-shaped encapsulated organs. Urine is formed by nephrons, the functional unit of the kidney, and then flows through a system of converging tubules called collecting ducts. The collecting ducts join together to form minor calyces, then major calyces, which ultimately join near the concave portion of the kidney (renal pelvis). A major function of the renal pelvis is to direct urine flow to the ureter. Urine flows from the renal pelvis into the ureter, a tube-like structure that carries the urine from the kidneys into the bladder. The outer layer of the kidney is called the cortex and is a rigid fibrous encapsulation. The interior of the kidney is called the medulla. The medulla structures are arranged in pyramids.

Each kidney is made up of approximately one million nephrons. Each nephron includes the glomerulus, Bowman's capsule, and tubules. The tubules include the proximal convoluted tubule, the loop of Henle, the distal convoluted tubule, and the collecting duct. The nephrons contained in the cortex layer of the kidney are distinct from the anatomy of those contained in the medulla. The principal difference is the length of the loop of Henle. Medullary nephrons contain a longer loop of Henle, which, under normal circumstances, allows greater regulation of water and sodium reabsorption than in the cortex nephrons.

The glomerulus is the beginning of the nephron and is responsible for the initial filtration of blood. Afferent arterioles pass blood into the glomerular capillaries, where hydrostatic pressure pushes water and solutes into Bowman's capsule. Net filtration pressure is expressed as the hydrostatic pressure in the afferent arteriole minus the hydrostatic pressure in Bowman's space minus the osmotic pressure in the efferent arteriole. $\begin{matrix}Net Filtration Pressure = Hydrostatic Pressure \left(\begin{matrix}Afferent \\ Arteriole\end{matrix}\right) - Hydrostatic Pressure \left(Bowman′s Space\right) - Osmotic \\ Pressure \left(Efferent Arteriole\right)\end{matrix}$ (Equation 1)

The magnitude of this net filtration pressure defined by Equation 1 determines how much ultra-filtrate is formed in Bowman's space and delivered to the tubules. The remaining blood exits the glomerulus via the efferent arteriole. Normal glomerular filtration, or delivery of ultra-filtrate into the tubules, is about 90 ml/min/1.73m².

The glomerulus has a three-layer filtration structure, which includes the vascular endothelium, a glomerular basement membrane, and podocytes. Normally, large proteins such as albumin and red blood cells, are not filtered into Bowman's space. However, elevated glomerular pressures and mesangial expansion create surface area changes on the basement membrane and larger fenestrations between the podocytes allowing larger proteins to pass into Bowman's space.

Ultra-filtrate collected in Bowman's space is delivered first to the proximal convoluted tubule. Reabsorption and secretion of water and solutes in the tubules is performed by a mix of active transport channels and passive pressure gradients. The proximal convoluted tubules normally reabsorb a majority of the sodium chloride and water and nearly all glucose and amino acids that were filtered by the glomerulus. The loop of Henle has two components that are designed to concentrate wastes in the urine. The descending limb is highly water permeable and reabsorbs most of the remaining water. The ascending limb reabsorbs 25% of the remaining sodium chloride, creating a concentrated urine, for example, in terms of urea and creatinine. The distal convoluted tubule normally reabsorbs a small proportion of sodium chloride, and the osmotic gradient creates conditions for the water to follow.

Under normal conditions, there is a net filtration of approximately 14 mmHg. The impact of venous congestion can be a significant decrease in net filtration, down to approximately 4 mmHg. *See* Jessup M., The cardiorenal syndrome: Do we need a change of strategy or a change of tactics?, JACC 53(7):597-600, 2009 (hereinafter "Jessup"). The second filtration stage occurs at the proximal tubules. Most of the secretion and absorption from urine occurs in tubules in the medullary nephrons. Active transport of sodium from the tubule into the interstitial space initiates this process. However, the hydrostatic forces dominate the net exchange of solutes and water. Under normal circumstances, it is believed that 75% of the sodium is reabsorbed back into lymphatic or venous circulation. However, because the kidney is encapsulated, it is sensitive to changes in hydrostatic pressures from both venous and lymphatic congestion. During venous congestion, the retention of sodium and water can exceed 85%, further perpetuating the renal congestion. *See* Verbrugge et al., The kidney in congestive heart failure: Are natriuresis, sodium, and diuretics really the good, the bad and the ugly? European Journal of Heart Failure 2014:16,133-42 (hereinafter "Verbrugge"). Venous congestion may result from, for example, heart failure, sepsis, burns, and other primary morbidities affecting renal pressure gradients and nephron filtration.

Venous congestion can lead to a prerenal form of acute kidney injury (AKI). Prerenal AKI is due to a loss of perfusion (or loss of blood flow) through the kidney. Many clinicians focus on the lack of flow into the kidney due to shock. However, there is also evidence that a lack of blood flow out of the organ due to venous congestion can be a clinically important sustaining injury. *See* Damman, K., Importance of venous congestion for worsening renal function in advanced decompensated heart failure, JACC 17:589-96, 2009 (hereinafter "Damman").

Prerenal AKI occurs across a wide variety of diagnoses requiring critical care admissions. The most prominent admissions are for sepsis and Acute Decompensated Heart Failure (ADHF). Additional admissions include cardiovascular surgery, general surgery, cirrhosis, trauma, burns, and pancreatitis. While there is wide clinical variability in the presentation of these disease states, a common denominator is an elevated central venous pressure. In the case of ADHF, the elevated central venous pressure caused by heart failure leads to pulmonary edema, and, subsequently, to dyspnea, which necessitates the admission. In the case of sepsis, the elevated central venous pressure is largely a result of aggressive fluid resuscitation. Whether the primary insult was low perfusion due to hypovolemia or sodium and fluid retention, the sustaining injury is the venous congestion resulting in inadequate perfusion.

Hypertension is another widely recognized state that creates perturbations within the active and passive transport systems of the kidney(s). Hypertension directly impacts afferent arteriole pressure and results in a proportional increase in net filtration pressure within the glomerulus. The increased filtration fraction also elevates the peritubular capillary pressure, which stimulates sodium and water reabsorption. *See* Verbrugge.

Because the kidney is an encapsulated organ, it is sensitive to pressure changes in the medullary pyramids. The elevated renal venous pressure creates congestion that leads to a rise in the interstitial pressures. The elevated interstitial pressures exert forces upon both the glomerulus and tubules. *See* Verburgge. In the glomerulus, the elevated interstitial pressures directly oppose filtration. The increased pressures increase the interstitial fluid, thereby increasing the hydrostatic pressures in the interstitial fluid and peritubular capillaries in the medulla of the kidney. In both instances, hypoxia can ensue leading to cellular injury and further loss of perfusion. The net result is a further exacerbation of the sodium and water reabsorption creating a negative feedback. *See* Verbrugge, 133-42. Fluid overload, particularly in the abdominal cavity, is associated with many diseases and conditions, including elevated intra-abdominal pressure, abdominal compartment syndrome, and acute renal failure. Fluid overload can be addressed through renal replacement therapy. *See* Peters, C.D., Short and Long-Term Effects of the Angiotensin II Receptor Blocker Irbesartanon Intradialytic Central Hemodynamics: A Randomized Double-Blind Placebo-Controlled One-Year Intervention Trial (the SAFIR Study), PLoS ONE (2015) 10(6): e0126882. doi:10.1371/journal.pone.0126882 (hereinafter "Peters"). However, such a clinical strategy provides no improvement in renal function for patients with the cardiorenal syndrome. *See* Bart, B., Ultrafiltration in decompensated heart failure with cardiorenal syndrome, NEJM 2012;367:2296-2304 (hereinafter "Bart").
US2015157836A1, published on behalf of FORSELL PETER MATS on June 11, 2015 relates to a method and a device for draining body fluid.
WO9529716A1, published on behalf of INFLUENCE INC on November 9, 1995 relates to medical apparatus, and particularly to medical devices adapted to be implanted into a subject's body and to be magnetically-coupled to an external drive magnet.

In view of such problematic effects of fluid retention, devices and methods for improving removal of urine from the urinary tract and, for example, for increasing quantity and quality of urine output from the kidneys, are needed. A solution to this problem is provided in the appended claims.

### SUMMARY

A pump assembly according to the present disclosure is defined by the patent claims. A pump assembly is provided comprising: (a) a pump module, wherein at least a portion of the pump module is configured to be positioned within at least one of an interior portion of a ureter, an interior portion of a renal pelvis, or an interior portion of a bladder for providing negative pressure to at least one of the patient's ureter or kidney, the pump module comprising: a housing comprising an open proximal end, an open distal end, and a sidewall extending therebetween defining a flow channel for conducting fluid through at least one of the interior portion of the patient's ureter, the interior portion of the patient's renal pelvis, or the interior portion of the patient's bladder wherein at least a portion of the housing is configured to be positioned within at least one of the interior portion of the ureter, the interior portion of the renal pelvis, or the interior portion of the bladder; and a pump element at least partially positioned within the channel configured to draw fluid through the channel between the open distal end and the open proximal end of the housing; and (b) a control module coupled to the pump module, the control module being configured to direct motion of the pump element to control flow rate of fluid passing through the channel, the control module comprising a housing configured to be positioned within at least one of a second interior portion of the patient's ureter, a second interior portion of the patient's renal pelvis, a second interior portion of the patient's bladder, or a second interior portion of the patient's urethra.

### BRIEF DESCRIPTION OF THE DRAWINGS

These and other features and characteristics of the present disclosure, as well as the methods of operation, use, and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limit of the invention.

Further features and other examples and advantages will become apparent from the following detailed description made with reference to the drawings in which:
FIG. 1A is a schematic drawing of a urinary tract of a patient showing a pump assembly positioned in the ureter and bladder of the patient according to an example of the present disclosure;
FIG. 1B is an enlargement of a portion of FIG. 1A;
FIG. 1C is a schematic drawing of a urinary tract of a patient showing a pump assembly positioned in the renal pelvis and ureter of the patient according to another example of the present disclosure;
FIG. 1D is a schematic drawing of a urinary tract of a patient showing a pump assembly positioned in the bladder of the patient according to another example of the present disclosure;
FIGS. 2A and 2B are schematic drawings of a pump assembly according to an example of the present disclosure;
FIG. 3 is a schematic drawing of a pump assembly including a wire linkage between a pump module and a controller module thereof, according to an example of the present disclosure;
FIG. 4 is a schematic drawing of a pump assembly including anchor barbs extending radially outward from a sidewall thereof, according to an example of the present disclosure;
FIG. 5 is a schematic drawing a pump assembly including spiral retention barbs, according to an example of the present disclosure;
FIG. 6 is a schematic drawing of a pump assembly including an inlet conduit configured to be inserted into a patient's ureter, according to an example of the present disclosure;
FIG. 7 is a cross-sectional view of a portion of the pump assembly of FIGS. 2A and 2B taken along line 7-7;
FIG. 8 is a cross-sectional view of a portion of a pump assembly according to an example of the present disclosure;
FIG. 9 is a schematic drawing of a pump assembly including a deployable induction coil according to an example of the present disclosure;
FIG. 10 is a schematic drawing of the pump assembly of FIG. 9 deployed in a patient's urinary tract, according to an example of the present disclosure;
FIG. 11 is a schematic diagram of electronic components of the pump assembly of FIGS. 2A and 2B;
FIG. 12 is a schematic drawing of a system for inducing negative pressure in a patient's urinary tract comprising a pump assembly according to an example of the disclosure;
FIG. 13A is a schematic drawing of a delivery catheter for delivery of a pump assembly into a portion of a urinary tract of a patient, according to an example of the present disclosure;
FIG. 13B is a schematic drawing of the delivery catheter of FIG. 13A with portions of an elongated tube cut-away to show the pump assembly contained therein; and
FIG. 14 is a schematic drawing of a pump assembly including a pump module positioned in a patient's bladder according to an example of the present disclosure.

### DETAILED DESCRIPTION

As used herein, the singular form of "a", "an", and "the" include plural referents unless the context clearly states otherwise.

As used herein, the terms "right", "left", "top", and derivatives thereof shall relate to the invention as it is oriented in the drawing figures. For a device or system, the term "proximal" refers to a portion of the device or system nearest to the access site through which the device or system is inserted into the body. For an indwelling urinary tract pump, the proximal portion is the portion of the device or system nearest to the urethra. The term "distal" refers to the opposite end of a device or system from the proximal end and, for example, to the portion of the device or system that is inserted farthest into the patient's urinary tract. However, it is to be understood that the invention can assume various alternative orientations and, accordingly, such terms are not to be considered as limiting. Also, it is to be understood that the invention can assume various alternative variations and stage sequences, except where expressly specified to the contrary. It is also to be understood that the specific devices and processes illustrated in the attached drawings, and described in the following specification, are examples. Hence, specific dimensions and other physical characteristics related to the embodiments disclosed herein are not to be considered as limiting.

For the purposes of this specification, unless otherwise indicated, all numbers expressing quantities of ingredients, reaction conditions, dimensions, physical characteristics, and so forth used in the specification and claims are to be understood as being modified in all instances by the term "about." Unless indicated to the contrary, the numerical parameters set forth in the following specification and attached claims are approximations that can vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Numerical values may inherently contain certain errors resulting from a standard deviation found in their respective testing measurements.

Also, it should be understood that any numerical range recited herein is intended to include all sub-ranges subsumed therein. For example, a range of "1 to 10" is intended to include any and all sub-ranges between and including the recited minimum value of 1 and the recited maximum value of 10, that is, all subranges beginning with a minimum value equal to or greater than 1 and ending with a maximum value equal to or less than 10, and all subranges in between, e.g., 1 to 6.3, or 5.5 to 10, or 2.7 to 6.1.

As used herein, the terms "communication" and "communicate" refer to the receipt or transfer of one or more signals, messages, commands, or other type of data. For one unit or component to be in communication with another unit or component means that the one unit or component is able to directly or indirectly receive data from and/or transmit data to the other unit or component. This can refer to a direct or indirect connection that can be wired and/or wireless in nature. Additionally, two units or components can be in communication with each other even though the data transmitted can be modified, processed, routed, and the like between the first and second unit or component. For example, a first unit can be in communication with a second unit even though the first unit passively receives data and does not actively transmit data to the second unit. As another example, a first unit can be in communication with a second unit if an intermediary unit processes data from one unit and transmits processed data to the second unit. It will be appreciated that numerous other arrangements are possible.

Generally, the pump assemblies, systems and methods of the present disclosure can be used to introduce a negative pressure or a positive pressure in at least a portion of patient's urinary tract to establish a desirable pressure gradient or pressure differential across the filtration anatomy of the nephron, e.g., glomerulus, proximal tubules, and distal tubules. In some examples, the pump assemblies, systems and methods of the present disclosure can be used to provide negative pressure therapy or positive pressure therapy for treatment of medical conditions such as acute or chronic treatment of venous congestion resulting from, for example, heart failure, sepsis, burn, and other primary morbidities impacting renal pressure gradients and nephron filtration. Systems for providing negative pressure therapy are also disclosed in International Publication No. WO 2017/015351 entitled "Ureteral and Bladder Catheters and Methods for Inducing Negative Pressure to Increase Renal Perfusion" and International Publication No. WO 2017/015345 entitled "Catheter Device and Method for Inducing Negative Pressure in a Patient's Bladder".

In some examples, the pump assemblies, systems and methods disclosed herein can be used to treat and/or control fluid retention and venous congestion, which can contribute to conditions such as heart disease, acute kidney injury, and renal failure. For example, fluid retention and venous congestion are known to be central problems in the progression to advanced renal disease. Excess sodium ingestion coupled with relative decreases in excretion lead to isotonic volume expansion and secondary compartment involvement. While not intending to be bound by any theory, it is believed that applying a negative pressure to the bladder, ureter, and/or kidney(s) can offset the medullary nephron tubule reabsorption of sodium and water in some situations. Offsetting reabsorption of sodium and water can increase urine production, decrease total body sodium, and improve erythrocyte production. Since the intra-medullary pressures are driven by sodium and, therefore, volume overload, the targeted removal of excess sodium enables maintenance of volume loss. Removal of volume restores medullary hemostasis. Normal urine production is 1.48-1.96 L/day (or 1-1.4 ml/min).

Fluid retention and venous congestion are also central problems in the progression of prerenal Acute Kidney Injury (AKI). Specifically, AKI can be related to loss of perfusion or blood flow through the kidney(s). Accordingly, in some examples, the present invention facilitates improved renal hemodynamics and increases urine output for the purpose of relieving or reducing venous congestion generally, and for example in the treatment of AKI, heart failure and/or kidney disease. Further, it is anticipated that treatment and/or inhibition of AKI positively impacts and/or reduces the occurrence of other conditions, for example, reduction or inhibition of worsening renal function in patients with NYHA Class III and/or Class IV heart failure. Classification of different levels of heart failure is described in The Criteria Committee of the New York Heart Association, (1994), Nomenclature and Criteria for Diagnosis of Diseases of the Heart and Great Vessels, (9th ed.), Boston: Little, Brown & Co. pp. 253-256. Reduction or inhibition of episodes of AKI and/or chronically decreased perfusion may also be a treatment for Stage 4 and/or Stage 5 chronic kidney disease. Chronic kidney disease progression is described in National Kidney Foundation, K/DOQI *Clinical* Practice Guidelines for Chronic Kidney Disease: Evaluation, Classification and Stratification. Am. J. Kidney Dis. 39:S1-S266, 2002 (Suppl. 1.

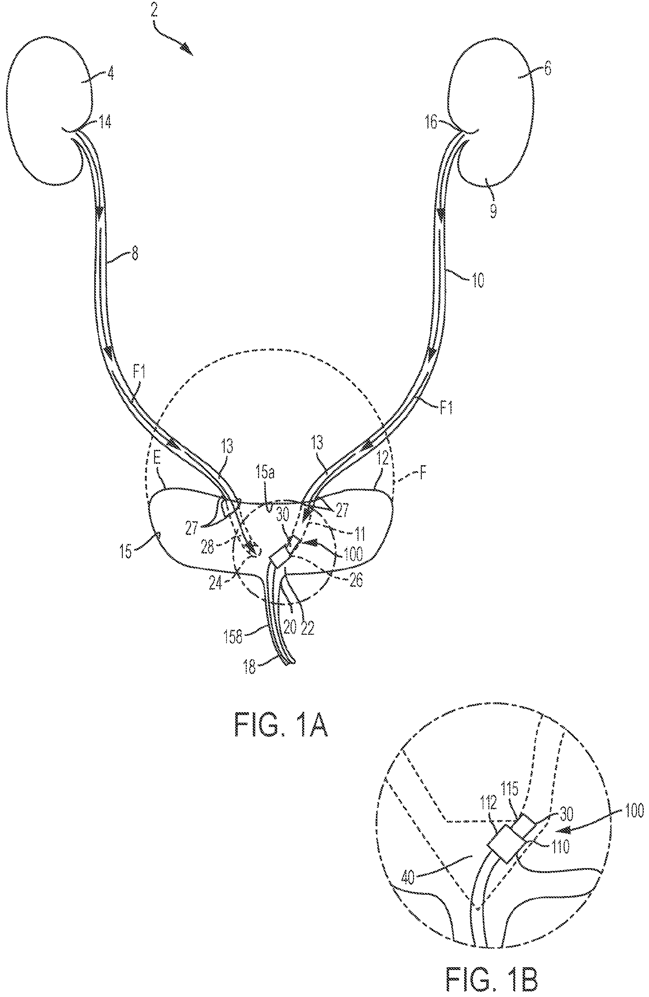

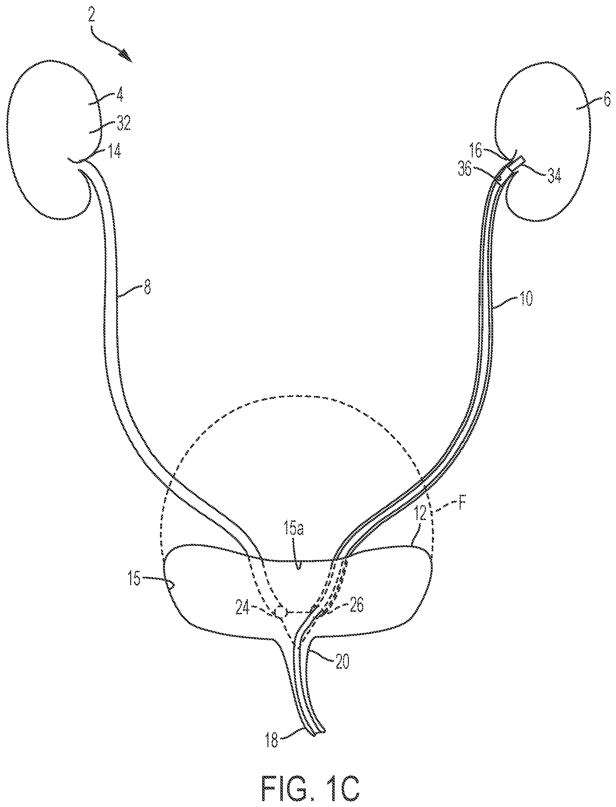

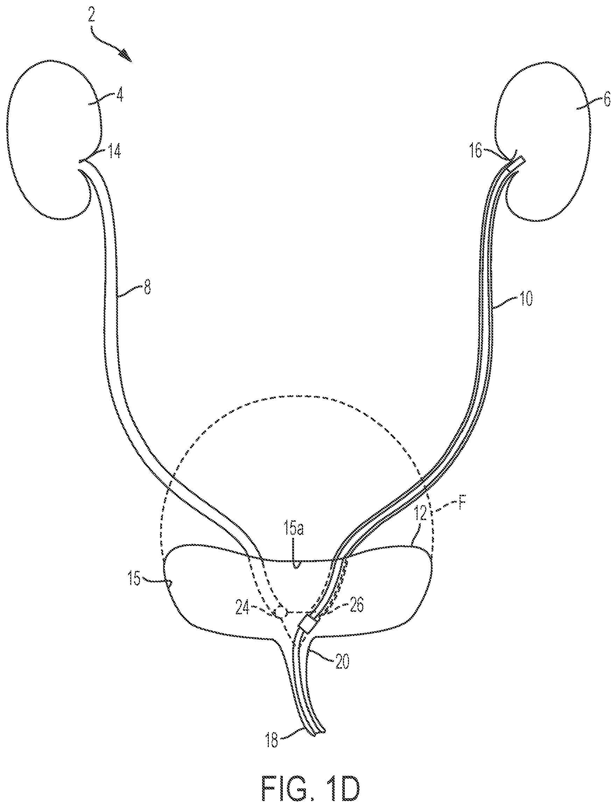

Referring now to FIG. 1, a pump or pump assembly according to the present disclosure which is configured or adapted to be placed within a urinary tract of a patient is disclosed herein. A urinary tract, shown generally as 2, of a patient comprises a patient's right kidney 4 and left kidney 6. The kidneys 4, 6 are responsible for blood filtration and clearance of waste compounds from the body through urine. Urine produced by the right kidney 4 and the left kidney 6 is drained into a patient's bladder 12 through tubules, namely, a right ureter 8 and a left ureter 10. For example, urine may be conducted through the ureters 8, 10 by peristalsis of the ureter walls, as well as by gravity. A distal portion 9 of the ureter 8, 10 and/or kidney 4, 6 known as the renal pelvis 14, 16 is a cornucopia-shaped structure extending between the ureter 8, 10 and kidney 4, 6. The ureters 8, 10 enter the bladder 12 through a ureter opening or orifice 24, 26. The bladder 12 is a flexible and substantially hollow structure adapted to collect urine until the urine is excreted from the body. The bladder 12 is transitionable from an empty position (signified by reference line E) to a full position (signified by reference line F). Normally, when the bladder 12 reaches a substantially full state, urine is permitted to drain from the bladder 12 to a urethra 18 through a urethral opening or sphincter 20 located at a lower portion of the bladder 12. Contraction of the bladder 12 can be responsive to stresses and pressure exerted on a trigone region 22 of the bladder 12, which is the triangular region extending between ureteral orifices 24, 26 and the urethral opening or sphincter 20. The trigone region 22 is sensitive to stress and pressure, such that as the bladder 12 begins to fill, pressure on the trigone region 22 increases. When a threshold pressure on the trigone region 22 is exceeded, the bladder 12 begins to contract to expel collected urine through the urethra 18.

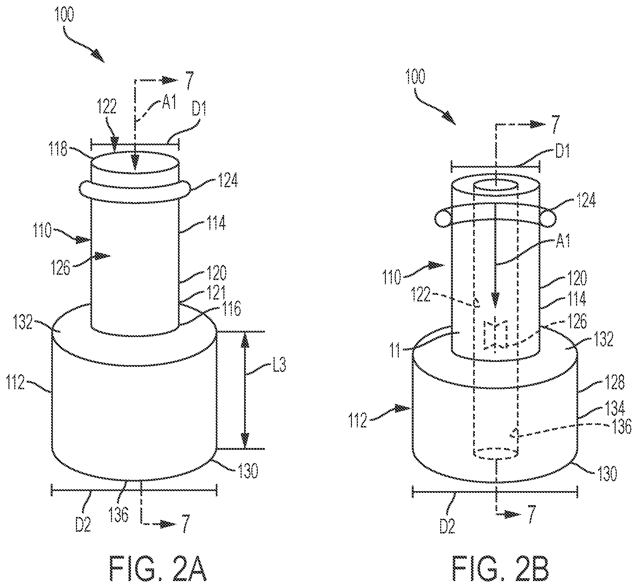

Referring now to Figs. 1A-D and 2A-2B, in some examples, the pump assembly, indicated generally at 100, comprises a pump module 110. At least a portion of the pump module 110 is configured to be positioned within at least one of an interior portion 28, 30 of a ureter 8, 10, an interior portion 32, 34 of a renal pelvis 14, 16, an interior portion 40 of a bladder 12, or an interior portion of a urethra 18 of a patient. For example, the pump module 110 can be configured to be positioned in a proximal portion 11 or a distal portion 9 of a patient's ureter 8, 10 and/or renal pelvis 14, 16. The pump module 110 can be used to provide negative or positive pressure, as desired, to at least one of the patient's ureter 8, 10 or kidney 4, 6.

An exemplary pump assembly 100 is shown in FIGS. 2A and 2B. The pump assembly 100 comprises a pump module 110 configured to be positioned within an interior portion 28, 30 (e.g., a proximal portion 11 or distal portion 9) of a ureter 8, 10 and/or an interior portion 32, 34 of a renal pelvis 14, 16 of a patient for providing negative or positive pressure to the patient's ureter and/or kidney, and a control module 112 configured to be implanted and/or deployed in a portion of the ureter 8, 10 and/or renal pelvis 14, 16 or, for example, elsewhere in a patient's urinary tract, for example, in the bladder 12 or the urethra 18.

### Pump module

In some examples, the pump module 110 comprises a housing 114. At least a portion of the housing 114 is configured to be positioned within an interior portion 28, 30 of the ureter 8, 10, an interior portion 32, 34 of the renal pelvis 14, 16, an interior portion 40 of the bladder 12, or an interior portion of the urethra 18. The housing 114 comprises an open proximal end 116, an open distal end 118, and a sidewall 120 extending therebetween, which defines a flow channel 122 for conducting fluid F1 through the interior portion 28, 30 of the patient's ureter 8, 10, the interior portion 32, 34 of the patient's renal pelvis 14, 16, the interior portion of patient's bladder 12, or the interior portion of the patient's urethra 18 (depending upon where the pump module is positioned) and to move the fluid into or through the patient's bladder 12 or urethra 18 to the exterior of the patient. The housing 114 can be formed from one or more suitable biocompatible materials, such as medical grade plastic (e.g., high-density polyethylene, polycarbonates, and silicone materials) and/or metal (e.g., surgical stainless steel).

In some examples, the housing 114 has a maximum external or outer diameter D1 of about 0.5 mm to about 3.0 mm, or about 2.0 mm to about 2.5 mm. The outer diameter D1 can be selected to correspond to an average ureter interior diameter, such that the pump module 110 fits snugly within the ureter.

In some examples, the pump module 110 may at least partially seal the ureter to inhibit bypass leakage of urine and/or to maintain negative pressure. In some examples, the outer cross-section of the housing 114 of the pump module 110 may be sized to fill the interior cross-section of the ureter. The engagement of the tissue of the ureter with the housing 114 at least partially seal the ureter to inhibit bypass leakage of urine and/or to maintain negative pressure. In one example, the housing may be substantially cylindrical and the outer diameter of the housing 114 may be equal to or larger than the interior diameter of the ureter. In some examples as shown in Fig. 2B, in order to facilitate formation of a suitable seal, a flexible and/or resilient elastomeric structure can be positioned about a portion of the exterior of the pump module to at least partially seal the ureter. For example, an annular seal 124 extending around the outer surface 121 of the sidewall 120 circumference of the pump module 110 can be attached to or positioned about a portion of the pump module 110 to form a seal between the housing 114 and the adjacent inner wall 13 of the ureter 8, 10. The seal 124 can be formed from one or more elastomeric biocompatible materials, such as silicone, polyurethane, polyolefin, or hydrogel such as alginate.

The housing 114 can be shaped as desired to facilitate positioning within the ureter, renal pelvis, bladder, or urethra and to accommodate the pump element (discussed below) and flow channel. In some examples, the housing 114 is a substantially cylindrical structure having substantially similar annular cross sections along its entire length. The housing 114 may have a diameter ranging from about 3 mm to about 8 mm or from about 4 mm to about 7 mm and a length ranging from about 5 mm to about 30 mm, from about 10 mm to about 25 mm, or from about 15 mm to about 20 mm. The pump module flow channel may have a diameter ranging from about 1 mm to about 6 mm or about 2 mm to about 5 mm. In other examples, the housing 114 can be tapered to facilitate positioning of distal portions of the housing 114 within the ureters 8, 10, the renal pelvis 14, 16, or the urethra 18. For example, a taper of about 0 to about 6 degrees may be used. In other examples, the housing 114 can have a non-circular cross section. For example, the cross section of the housing 114 can be a square, rectangle, another polygonal shape, or combinations thereof. In some examples, the housing can have a generally smooth outer surface to improve patient comfort.

As discussed in further detail below, to induce negative or positive pressure, the pump module 110 comprises a pump mechanism or element 126 (shown in Fig. 2B) which, while positioned within an interior portion 28, 30 of the ureter 8, 10, an interior portion 32, 34 of the renal pelvis 14, 16, an interior portion 40 of the bladder 12, or an interior portion of the urethra 18 is continuously or periodically activated to draw fluid into a flow channel 122 of the pump module 110, thereby inducing negative or positive pressure in the ureters 8, 10 and/or kidneys 4, 6. The pump element 126 can be at least partially positioned within the channel 122 such that, when activated, draws fluid through the channel 122 between the open distal end 118 and the open proximal end 116 of the housing in the direction of arrow A1. The pump element 126 can operate for a predetermined period of time, for example for a certain period each day, or it can operate continuously. The period of time of pump operation can vary, as desired. The pump element 126 can include different types of molded or machined parts, as are known in the art, including impellers, screw threads, pistons, one-way valves, check valves, and similar structures for drawing fluid through the pump module, as will be described herein. In some examples, the pump element 126 comprises a piezoelectric film or surface which transitions from an extended configuration to a retracted configuration to draw fluid through the pump, such as is described below.

When actuated, the pump module 110 draws fluid F1 (e.g., urine) from the kidney(s) 4, 6 and ureter(s) 8, 10 and moves the fluid F1 to the bladder 12 or through the bladder to outside of the patient's body, thereby inducing a negative pressure in the urinary tract. The rotation or actuation of the pump element 126 can be reversed to provide positive pressure, as needed.

In some examples, fluid F1 is expelled by the pump module 110 into the bladder 12. In other examples, fluid F1 can be conducted through an outlet line 158, such as a tube or conduit, thorough the inside of the urethra 18 and outside the body. The fluid F1 can be collected in a fluid collection container (not shown) located outside of the patient's body. The pump module may be configured to deliver negative pressure in a range of 0 to about 20 kPa (150 mmHg), or about 667 Pa (5 mmHg) to about 13.33 kPa (100 mmHg), or about 1.333 kPa (10 mmHg) to about 6.666 kPa (50 mmHg). The pump module may be configured to intermittently deliver positive pressure in a range of about 0 to about 20 kPa (150 mmHg), or about 133 Pa (1 mmHg) to about 13.33 kPa (100 mmHg), or about 133 Pa (1 mmHg) to about 6.666 kPa (50 mmHg). The pump module 110 can be configured to provide a volumetric fluid flow rate between 0 and about 3.5 mL/min, between about 0.2 mL/min and about 2.5 mL/min, or between about 0.4 mL/min and about 1.25 mL/min. Generally, the amount of negative or positive pressure delivered by the pump and/or volumetric flow rate is determined from pump operating parameters (e.g., the pump module is set to deliver a predetermined negative pressure or to extract fluid at a predetermined flow rate). However, in some examples, the pump module can comprise pressure sensors for directly or indirectly measuring negative and/or positive pressure exerted on the ureter and/or kidneys by the pump module and/or flow rate sensors for measuring fluid volume drawn through the pump module. As described herein, negative or positive pressure may be applied continuously or intermittently pulsed to drive continuous or pulsatile flow.

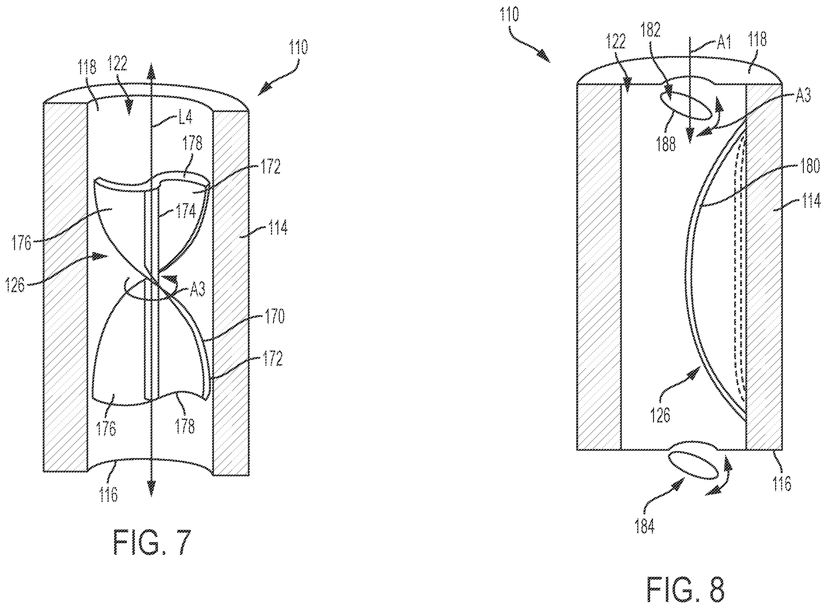

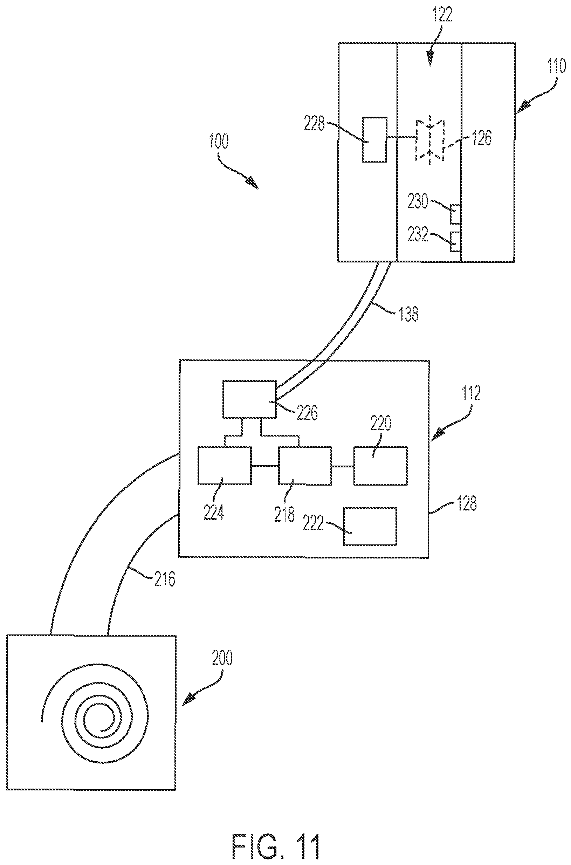

As described herein, the pump element 126 can be, for example, a micro-electrical mechanical component as is known in the art, for example, an Abiomed^{®} Impella^{®} pump or a piezoelectric pump such as those manufactured by Dolomite. Other fabrication techniques for producing components of a pump module 110 configured for insertion in the ureter 8, 10, the renal pelvis 14, 16, or the urethra 18 can comprise, for example, injection molding, three-dimensional printing, metal stamping, and similar fabrication techniques, as are known in the art. For example, as shown in FIGS. 7 and 8, the pump element 126 may comprise an impeller 1 and/or a deformable, a moveable, and/or an expandable piezoelectric element 180. As described in greater detail below with respect to FIG. 11, in some examples, the pump element 126 can be operatively connected to electrical components including a motor (e.g., drive mechanism 228), a power source (e.g., battery 226 and/or induction coil 210), and an on/off switch or controller 218, as well as to different types of optional sensors for measuring pump operating parameters and/or physiological information for the patient, as discussed below.

### Pump element and associated electronic components

Exemplary embodiments of the pump element 126 of the pump module 110 are shown in FIGS. 7 and 8. As discussed previously, the pump element 126 can be positioned at least partially within the channel 122 defined by the pump module housing 114. When activated, the pump element 126 draws fluid, such as urine produced by the kidney, into the channel 122 through the open distal end 118 of the housing 114 and expels the fluid through the open proximal end 116 of the housing 114. In some examples, the pump element 126 may also propel fluid through the channel 136 defined by the control module housing 128 (shown in FIG. 2B) and into the patient's bladder, or through a tube through the bladder and urethra and external to the patient's body.

As shown in FIG. 7, in some examples, the pump element 126 comprises a rotatable impeller 170 positioned within the channel 122. The impeller 170 can be made from various medical-grade materials which are sufficiently strong and rigid to rotate for a prolonged duration without deforming or bending. For example, the impeller 170 can be formed from a metal material, such as surgical stainless steel, and/or from a rigid plastic material, such as polycarbonate. For example, the impeller 170 can comprise two or more blades 172 mounted to and positioned to rotate about a central rotor 174 in a direction of arrow A3. The impeller 170 can have 2 to 4 blades. The blades 173 can have a length of about 8 mm to about 14 mm or about 10 to about 12 mm and a width of about 2 mm to about 3 mm. The clearance between the blades 172 can be about 0.02 mm to about 1 mm or about 0.5 mm to about 0.8 mm. As shown in FIG. 7, the rotor 174 may extend longitudinally through the channel 122 along a central longitudinal axis L4 thereof. The blades 172 may comprise a straight or curved surface 176 configured to contact fluid passing through the channel 122. In some examples, the blades 172 may also be able to rotate about the rotor 174 in an opposite direction to apply positive pressure to the ureter and/or kidney if desired. The blades 172 can have any suitable shape, which when rotated, is capable of drawing fluid through the channel. For example, as shown in FIG. 7, edges 178 of the blades 172 may have a straight, curved or "S"-shaped configuration. As previously discussed, the pump element 126 and impeller 170 can be operatively connected to the drive mechanism or electric motor which, when activated, causes the blades 172 to rotate as described herein.

As shown in FIG. 8, another exemplary pump element 126 comprises a piezoelectric diaphragm 180 configured to transition between a contracted position (shown by dashed lines in FIG. 8) and an expanded position (shown by sold lines in FIG. 8), in which the piezoelectric diaphragm 180 expands into the channel 122 to restrict flow through the channel 122 and reduce a volume and cross-sectional area of the channel 122. The piezoelectric diaphragm 180 can be formed from a thin flexible conductive film, such as a polymer and/or elastomeric film, as is known in the art or from stainless steel. The piezoelectric diaphragm 180 can be electronically coupled to a drive mechanism, such as a signal generator or power source for activating the piezoelectric diaphragm 180. For example, the diaphragm 180 can be activated by passing an electric signal generated by the signal generator or power source through the conductive film of the diaphragm 180 to cause the diaphragm 180 to transition to the extended position. During use, one side of the piezoelectric diaphragm 180 is exposed to the fluid and the drive mechanism is located on the unexposed side of the piezoelectric diaphragm 180. The pump element 126 further comprises valves 182, 184, such as one-way check valves, positioned at the open distal end 118 and open proximal end 116 of the channel 122, respectively, as shown in FIG. 8. The check valves 182, 184 can be conventional one-way valves configured to restrict backflow of fluid, as is known in the art. Exemplary one-way check valve mechanisms can include, for example, a flexible flap or cover, ball valve, piston valve, or similar mechanism.

In operation, fluid is drawn into channel 122 through a distal valve 182 by deflation of the piezoelectric diaphragm 180, as shown by arrow A1 in FIG. 8. For example, a flap 188 of the distal valve 182 may pivot in a direction of arrow A3 to an open position to permit fluid to pass therethrough. As a result of negative pressure produced by deflation or collapsing of the diaphragm 180, the proximal valve 184 is forced to close to prevent backflow of fluid. Once the diaphragm 180 is deflated or collapses by a predetermined amount, motion of the diaphragm 180 is reversed by applying the electric signal to the conductive film. As the diaphragm 180 expands, the distal valve 182 closes to prevent fluid backflow and fluid is expelled from the channel 122 through the open proximal valve 184 through the open proximal end 116 of the housing, into the lower ureter portion 11 and through the urethra 18.

### Control module

The pump assembly 100 further comprises a control module 112 configured to be positioned within at least one of a second interior portion of the patient's ureter 8, 10, a second interior portion of the patient's renal pelvis 14, 16, a second interior portion of the patient's bladder 12, or a second interior portion of a patient's urethra. As used herein, a module can refer to a device in wired or wireless communication with one or more other modules or devices, thereby forming a patient treatment system. In some examples, modules can be portions of a single device or assembly or multiple devices or assemblies and, for example, can be enclosed in a single device housing or multiple housings. In other examples, a module refers to processing circuitry which executes instructions and performs functions based on the executed instructions. In that case, the same processing components may perform functions of different modules. For example, a single controller or microprocessor may be configured to perform both functions of the pump module 110, including actuating and ceasing operation of a pump mechanism or pump element 126, and of the control module 112, such as receiving and processing data transmitted from remote devices.

In some examples, the control module comprises electronic circuitry, such as a controller or microprocessor comprising computer readable memory comprising instructions, that when executed, control pump operating parameters (e.g., flow rate, operating speed, operating duration, etc.). For example, the controller or processor can be configured to output instructions to the pump module to cause the pump module to turn on, turn off, or adjust operating speed. The control module can also comprise one or more communications interfaces for communicating instructions to the pump module and for communicating information about treatment provided to the patient and measured patient parameters to a remote device or data collection facility. For example, the communications interface may be configured to wirelessly transmit data about a patient or treatment provided to a patient to a patient care facility for inclusion in a patient health record.

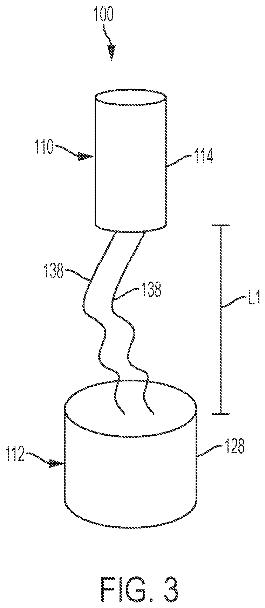

The pump module 110 and control module 112 may be integrally formed or directly connected as shown, for example, in FIGS. 2A and 2B. In other examples, separate pump and control modules can be connected by a wireless or wired connection, as shown in FIG. 3. In some examples, the wires extending between the pump module and the control module may extend a substantial portion of the length of the ureter, so that the pump module can be positioned within the renal pelvis region, and the control module can be positioned in the patient's bladder. In other examples, the pump module may be in wireless communication with the control module, which can be spaced apart from the pump module. For example, a remote control device 310, such as a device positioned outside of the patient's body, can be used to control the pump module 110.

The control module 112 is operatively connected to and/or in communication with components of the pump module 110 including the pump element 126 to direct motion of the pump element 126 to control the flow rate of fluid F1 passing through the interior portion of the patient's ureter 8, 10, the patient's renal pelvis, the patient's bladder 12, or the patient's urethra 18.

In some examples, the control module 112 comprises a housing 128. At least a portion of the housing 128 is configured to be positioned within the interior portion 28, 30 of the ureter 8, 10, the interior portion 32, 34 of the renal pelvis 14, 16, the internal portion of the bladder 12, the internal portion of the urethra 18\, or elsewhere in the urinary tract 2. The housing 128 comprises a distal end 132, a proximal end 130, and a sidewall 134 extending therebetween. In some examples, the length of the control module 112 can vary, for example the control module 112 can have a length L3 of between about 1 cm to about 5 cm or about 2 cm to about 4 cm. The control module 112 can have a maximum outer diameter D2 which, in some examples, is greater than the maximum outer diameter D1 of the pump module housing 114. The diameter D2 of the control module 112 can vary, for example the control module 112 can have a diameter of between about 5 mm to about 20 mm or about 10 mm to about 15 mm.

The control module housing 128 can be shaped as desired to facilitate positioning within the ureter, the renal pelvis 14, 16, the bladder 12, or the urethra 18, and to accommodate the control module and the flow channel (if present). In some examples, the control module housing 128 is a substantially cylindrical structure having substantially similar annular cross sections along its entire length. In other examples, the control module housing 114 can be tapered to facilitate positioning of distal portions of the control module housing 128 within the ureters 8, 10, the bladder 12, or the urethra 18. For example, a taper of about 0 to about 6 degrees may be used. In other examples, the control module housing 128 can have a non-circular cross section. For example, the cross section of the control module housing 128 can be a square, rectangle, or another polygonal shape.

In some examples, the control module housing 128 is a generally cylindrical structure. The control module housing 128 can optionally comprise a flow channel 136 therethrough. The flow channel 136 can have an inner diameter ranging from about 1 mm to about 6 mm or about 2 mm to about 5 mm. The shape of the interior of the flow channel 136 can have any shape as desired, and in some examples can be generally cylindrical to facilitate flow therethrough. The control module housing 128 can be formed from a similar biocompatible metal or plastic material as the pump module housing 114 described above. In general, the maximum outer diameter D2 can be sufficient to position the control module 112 within the bladder of a patient and, as such, can be wider than the diameter of the ureteral orifice 24, 26 (shown in FIG. 1) and the interior diameter of the ureter so that the control module 112 remains in the bladder and is not drawn into the ureter along with the pump module 110.

The control module 112 further comprises electronic circuitry for operating the pump element 126, including components for controlling and adjusting pump flow rate, negative and/or positive pressure generated, power usage, and other operating parameters. Exemplary electronic components of the pump assembly 100, including of the control module 112, are shown in FIG. 11 and are described below in detail.

As shown in FIGS. 2A and 2B, in some examples, the pump module 110 and the control module 112 can be integrally formed, such that the respective housings 114, 128 are directly connected to one another. For example, as shown in FIGS. 2A and 2B, the proximal end 116 of the pump module 110 is connected to and/or integrally formed with the distal end 132 of the control module 112. In that case, the channel 122 of the pump module 110 is directly connected to and in fluid communication with the channel 136 of the control module 112, such that fluid F1 is drawn from the ureter 8, 10, the renal pelvis 14, 16, the bladder 12, or the urethra 18 through the flow channel 122 of the pump module 110 and the flow channel 136 of the control module 112, and expelled from the open proximal end 130 of the control module 112 into the patient's bladder or through tubing to conduct the fluid through the bladder 12 and urethra 18 to outside of the patient's body.

As shown in FIG. 3, in another example of a pump assembly 100, the control module housing 128 is separate from the pump module housing 114. In that case, as shown in FIG. 3, the respective modules 110, 112 are connected via a wireless or wired connection formed by one or more wires 138 extending between the respective modules 110, 112. In some examples, the wires 138 are coated with a suitable biocompatible sheath or coating to provide suitable insulation and to facilitate insertion and/or removal of the wires 138 from the urinary track. For example, polymer coatings, such as polyvinyl chloride, polytetrafluoroethylene (PTFE), latex, or silicone may be used. In this example, the control module housing can optionally include the flow channel 136.

The wires 138 can be configured to conduct electronic signals between the modules 110, 112 including, for example, operating instructions from the control module 112 to the pump element 126 of the pump module 110 to control or adjust operating speed and/or to actuate or cease operation of the pump element. Operating parameters and/or information sensed by electronic components of the pump module 110 may be transmitted to the control module 112 via the wired connection for processing, analysis, and/or to be transmitted from the control module 112 to a remote source. In some examples, the wires 138 between the respective modules 110, 112 are rather short in length, meaning that the pump module 110 is configured to be positioned in a proximal portion of the ureter 8, 10, near the ureteral orifice 24, 26 into the bladder 12. In other configurations, the wires 138 are about the length of the ureter, meaning that the pump module 110 can be positioned in the renal pelvis 14, 16 and/or kidney 4, 6, while the control module 112 can be positioned in the ureter 8, 10 and/or the bladder 12. For example, the wires 138 may have a length L1 of about 1 cm to about 35 cm, or about 15 cm to about 25 cm, since the average ureter length of an adult is about 25 cm to about 30 cm.

### Power source

In some examples, the control module 112 further comprises an internal power source or external power source 200 for providing power for the electronic circuitry of the control module 112 and pump element 126 or mechanism of the pump module 110. The power source can be a disposable or rechargeable battery, which, in some examples, may be recharged, for example, using inductive power transfer through a small induction coil deployed, for example, in the bladder. The induction coil may be configured to generate power when exposed to an electromagnetic field generated by a remote device outside of the patient's body. For example, the remote device can be a computerized device, such as a smart phone or tablet PC. In other examples, the remote device can be a non-computerized device including circuitry for generating the electromagnetic field. In one example, a blanket including field generating electromagnetic circuitry can be wrapped around the patient while he/she sleeps. The field generating circuitry can induce the coil to generate power for the entire night or at least until the rechargeable battery is fully charged. Since the patient is less likely to move while sleeping than when awake, it is likely that the portion of the blanket which produces the electromagnetic field remains in close proximity to the pump assembly for a substantial period of time.

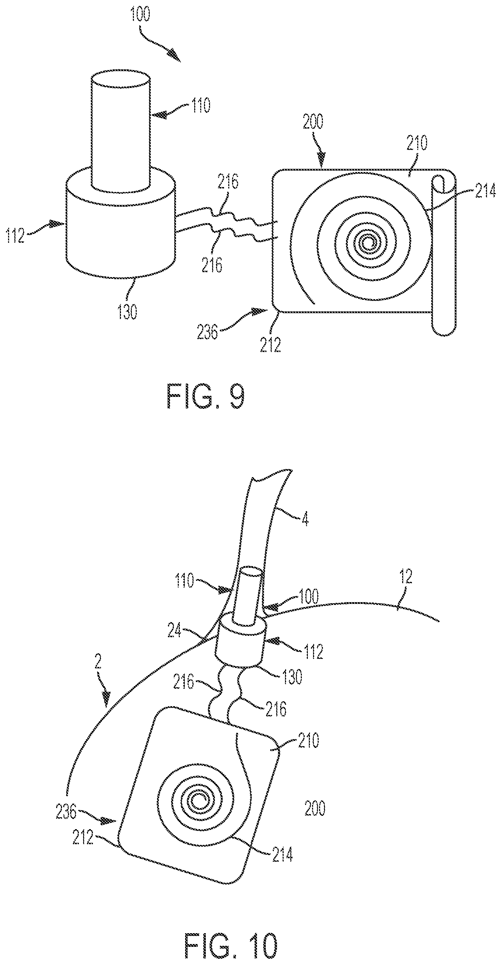

In some examples, the pump assembly 100 further comprises a power source 200, as shown in FIGS. 9 and 10, comprising an induction coil 210 electronically coupled to the pump module 110 and/or to the control module 112. Induction coils for near field wireless energy transfer are known and are used, for example, to charge portable low-power electronic devices such as cell phones, laptop computers, small electrical appliances, and power tools. An exemplary induction coil is the eCoupled system developed by Fulton Innovation, which is described, for example, in U.S. Patent No. 6,975,198 entitled "Inductive Coil Assembly". Other known or later developed induction systems, which can be positioned in a patient's body and used to generate sufficient power to operate a microelectromechanical device or system may also be used within the scope of the present disclosure.

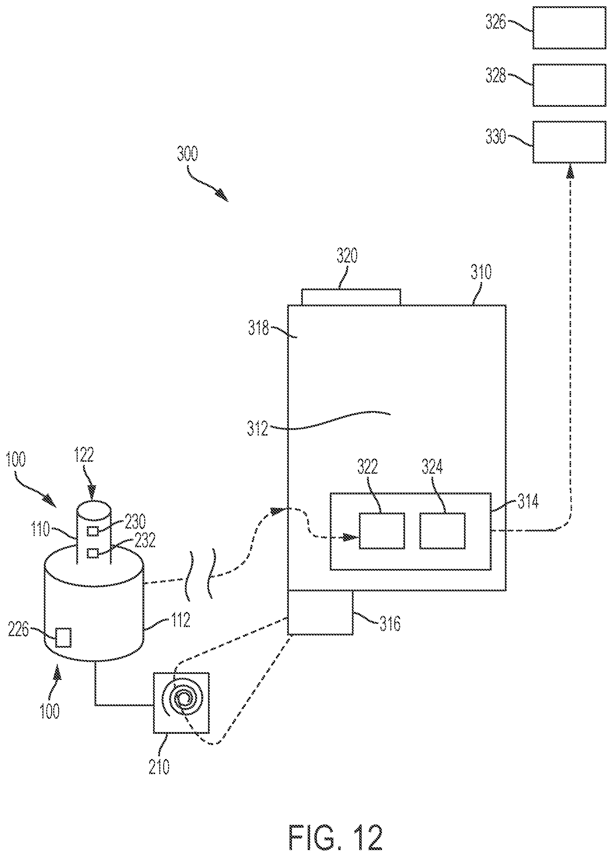

As described herein, the induction coil 210 generates and provides power to the pump assembly 100 to operate the pump module 110 and control module 112. For example, power produced by the induction coil 210 can be used to recharge a rechargeable battery, to provide power for sensors disposed in the pump module 110, and/or for wireless data transmission between the pump assembly 100 and external devices. In some examples, as discussed herein, the induction coil 210 generates power when exposed to an electromagnetic field produced by another device. For example, the electromagnetic field can be generated by a remote control device 310 (shown in FIG. 12) positioned outside the patient's body. The remote control device 310 can be worn in a holster, carrying case, fanny pack, or pocket, for example, and positioned such that the remote device is held flat against the body and as close as possible to the induction coil 210.

In some examples, shown in FIGS. 9 and 10, the induction coil 210 comprises a flexible sheet 212, such as a polymer sheet, and a conductive wire 214 embedded or attached to the flexible sheet 212. For example, the wire 214 can be attached to the flexible sheet 212 in a spiral pattern, a zig-zag pattern, or any other suitable pattern. The induction coil 210 can be connected to the pump assembly 110 by one or more wires or cables 216. For example, the coil 210 can be connected to the control module 112 through cables 216 extending from the proximal end 130 of the control module 112.

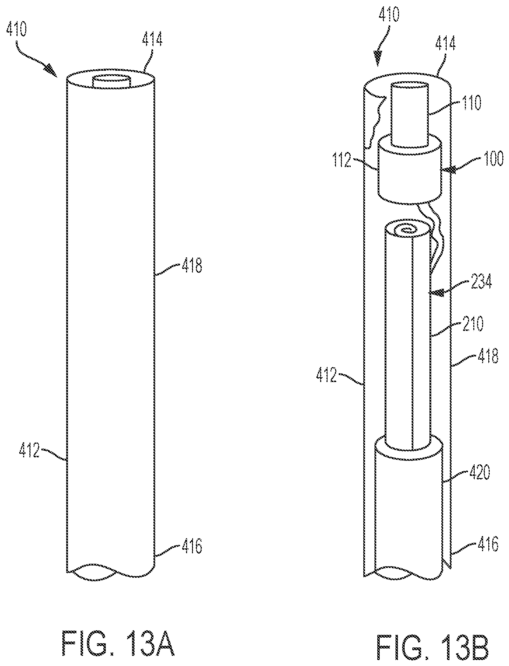

In some examples, the flexible sheet 212 is transitionable between a rolled configuration 234 (as shown in FIG. 13B) and an un-rolled or deployed configuration 236 (shown in FIGS. 9 and 10). In some instances, the flexible sheet 212 may be configured to deploy automatically. For example, the sheet 212 may be biased to naturally unroll when it is released from a deployment catheter 410 (shown in FIGS. 13A and 13B). In other examples, the coil 210 may include a manual release mechanism such as a release button or trigger wire. When a user presses the release button or pulls the trigger wire, a latching mechanism for maintaining the coil 210 in the rolled configuration releases allowing the flexible sheet 212 to unroll, thereby transitioning the sheet 212 to the deployed configuration.

The induction coil 210 can be positioned at any convenient position within the patient's urinary tract 2. For example, as shown in FIG. 10, the induction coil 210 can be operatively connected to the control module 112 and is positioned in the patient's bladder 12 at a position proximal to the control module 112. Alternatively, the induction coil 210 may be positioned in the abdominal cavity, outside of the bladder, in the peritoneal cavity, in any other convenient location in vivo, or external to the patient.

### Pump assembly electrical components

Exemplary electronic components of the pump assembly 100 are shown in the schematic diagram of FIG. 11. As previously described, the pump assembly 100 comprises the pump module 110, control module 112, and power source, such as induction coil 210 or battery 226 (shown in FIG. 11). The induction coil 210 can be operatively coupled to the control module 112 by cables 216 for proving power to the control module 112.

The control module 112 comprises a controller 218 and associated transitory or non-transitory computer readable memory 220. The controller 218 can comprise, for example, one or more general-purpose microprocessors configured to receive and implement instructions for operating the pump by, for example, communicating with the pump module 110 to actuate or cease operation of the pump element 126 and/or to adjust an operating speed to control negative and/or positive pressure delivered to the patient.

In some examples, the controller 218 can be configured to control communication between the pump assembly 100 and one or more remote control devices 310 located external to the patient. In that case, the control module 112 may further comprise a communications interface 222 comprising, for example, a wireless transmitter or antenna. The communications interface 222 can be configured to receive instructions from a remote source (e.g., remote control device 310) and to emit signals controlling operation of the pump element based on the received instructions.

The control module 112 further comprises power distribution and management circuitry 224. As shown in FIG. 11, the power management circuitry is electrically coupled to the induction coil 210. The power distribution circuitry 224 can be configured to receive power generated by the induction coil 210 and to control distribution of the generated power to other system components.

In some examples, the control module 112 may further comprise a battery 226, such as a rechargeable battery, operatively connected to the controller 218 and power distribution circuitry 224. The battery 226 can be recharged from power generated by the induction coil 210. At times when power is not being generated by the induction coil 210, system components can continue to operate with power provided by the battery 226. The battery 226 can be any battery which is small enough to fit within the control module housing 128 and which has been approved for use *in vivo.* For example, batteries used in pacemakers and similar implanted devices may be appropriate for use with the pump assembly 100 described herein.

As previously described, electronic components of the control module 112 including the controller 218 are in electronic communication with electrical components of the pump module 110 through, for example, connecting wires 138 (shown in FIGS. 3, 9, and 11) or by another suitable electronic connection. Power generated by the induction coil 210 can be provided to electronic components of the pump module 110 through the wires 138. Additionally, operating instructions generated by the controller 218 can be provided to components of the pump module 110 to control pump operating parameters. In a similar manner, information collected or generated by components of the pump module 110 can be communicated to the controller 218 for further processing and/or to be transmitted wirelessly to a remote device.

As shown in FIG. 11, the pump module 110 comprises the pump element 126 and associated electronic components. For example, the pump module 110 can comprise a drive mechanism 228, such as an electric motor or signal generator, operatively connected to the pump element 126. A variety of different drive mechanisms can be used in connection with the pump module 110 depending on the type of pump element 126 being used. For example, for an impeller-type pump arrangement (as shown in FIG. 7), the drive mechanism 228 may comprise an electric motor which causes the impeller to rotate. In other examples, the drive mechanism 228 can comprise electromagnetic elements disposed around the impeller 170, which turn on and off in a predetermined pattern to cause the impeller to rotate at a desired speed. If the pump element 126 is a piezoelectric element 180, then the drive mechanism 228 can comprise a signal generator for generating electric current to transition the piezoelectric element between the contracted and expanded states.

In some examples, the pump module 110 can further comprise one or more sensors (e.g., pump sensors 230 and physiological sensors 232) positioned within the flow channel 122 of the pump module 110 for measuring information about pump operating conditions and/or about fluid passing through the channel 122. For example, pump sensors 230 can comprise flow sensors for confirming that fluid is passing through the channel 122 and/or for measuring flow rate. Pump sensors 230 can also comprise sensors for measuring an amount of negative and/or positive pressure generated or a pump impeller rotation speed. Physiological sensors 232 can comprise one or more sensors for measuring information about fluid passing through the channel 122 to determine information about the physiological condition of the patient. Exemplary physiological sensors 232 can comprise, for example, capacitance and/or analyte sensors for measuring information representative of chemical composition of generated urine, pH sensors for measuring acidity of urine, or temperature sensors for measuring urine temperature.

### Retention members

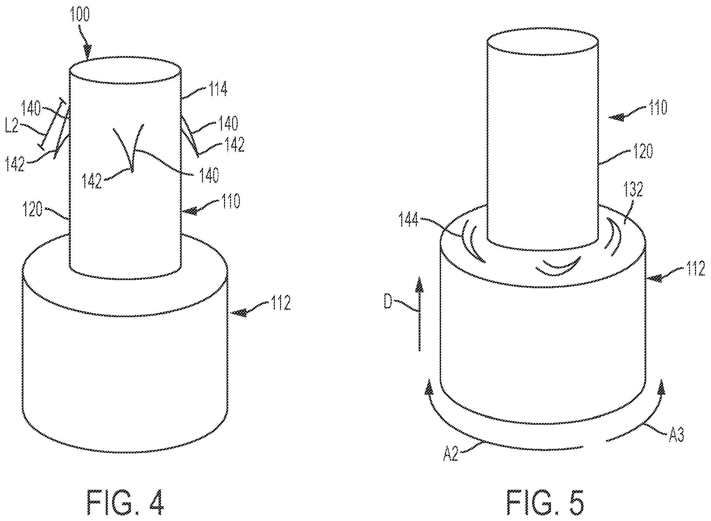

In some examples, and as shown in FIG. 4, the pump assembly 100 can further comprise one or more retention members, for example retention barbs 140, and/or spiral barbs 144, for maintaining position of the pump module 110 and/or control module 112 within the patient's urinary tract 2. In some examples, the pump module housing 114 and/or control module housing 128 comprises one or more retention members extending from the sidewall for releasably attaching a portion of the pump module housing 114 to at least one of the interior portion of the ureter, the interior portion of the renal pelvis, the interior portion of the bladder, or the interior portion of the urethra of a patient. The retention members are retractable to permit removable of the pump module from the ureter, renal pelvis, the bladder, or the urethra.

In some examples, retention members or retention barbs 140, 144 can be formed in any suitable pattern or arrangement including straight ridges, curved ridges, sharpened protrusions, fish hooks, and combinations thereof. For example, the retention barbs 140 can be deployable and retractable. In that case, the barbs 140 may be in a retracted position as the pump assembly 100 is being advanced through the urinary tract. Once the pump assembly 100 is advanced to a desired position, the barbs 140 are deployed to engage portions of the ureter, renal pelvis, bladder or urethra wall to hold the pump assembly 100 in a desired position. For example, as shown in FIG. 4, an exemplary pump assembly 100 comprises one or more barbs 140 extending radially outward from the sidewall 120 of the pump module 110. The barbs 140 may be flat so that the barbs 140 can be compressed against the sidewall 120 of the housing 114 of the pump module 110 to disengage the tissue of the ureter, renal pelvis, bladder or urethra during removal of the pump assembly 100. Alternatively, the barbs 140 can have any suitable configuration or cross sectional shape including a triangle, circle, semi-circle, rectangle, trapezoid, or polygon. The barbs 140 may have a longitudinal length L2 that may be at least about 0.25 mm or at least about 0.45 mm and may be up to about 3.0 mm, up to about 2.5 mm, or up to about 1.5 mm. The barbs 140 may have a width or diameter of about 1.0 mm or less, about 0.8 mm or less, or about 0.5 mm or less. Prior to insertion, the barbs 140 may extend a maximum distance from the sidewall 120 of the pump module 110 a distance of about 1.0 mm or less, about 0.8 mm or less, or about 0.5 mm or less. The barbs 140 can be formed from a semi-rigid or rigid material suitable for maintaining positioning of the pump module 110 in the urinary tract. For example, the barbs 140 can be formed from metal (e.g., surgical stainless steel) or plastic. The barbs 140 may comprise a sharpened tip 142 for pressing against and slightly encroaching into the ureter, renal pelvis, bladder, or urethra wall to maintain positioning of the pump module 110, without perforating the ureteral wall. Ureteral wall thickness is about 0.05 mm to 0.1 mm, so the tips 142 of the barbs 140 should encroach into the ureter, renal pelvis, bladder, or urethra wall by less than that amount.

In some examples, the barbs 140 are retractable. For example, barbs 140 can be biased in a radially outward direction toward the ureter, renal pelvis, bladder, or urethra wall, but configured to retract against the sidewall 120 of the pump module housing 114 when the pump assembly 100 is being advanced through a deployment catheter and into the patient's ureter, renal pelvis, bladder, or urethra. Once in a deployed position, the barbs 140 may be configured to extend radially outward to a deployed configuration, as shown in FIG. 4. In other examples, the barbs 140 can be extended or retracted by a manually activated triggering mechanism. For example, a user may press a retraction button or pull on a triggering wire to remove a radial biasing force from the barbs 140 to cause the barbs 140 to retract. After the barbs 140 retract, the pump assembly 100 can be safely and easily removed from the urinary tract. For example, the pump module 110 and control module 112 can be removed through the bladder and the urethral sphincter and then from the body through the urethra.

In some examples, the control module 112 comprises one or more retention members or barbs 144 for anchoring or retaining the control module to an interior surface of the bladder wall, in addition to or in lieu of the retention members on the pump module. The barbs 144 can be similar to those discussed above for barbs 140. For example, as shown in FIG. 5, retention barbs 144, such as spiral retention barbs, extend in a distal direction D from the distal end 132 of the control module 112. When positioned in the bladder 12, the barbs 144 are configured to contact portions 27 of the bladder wall 15 around the ureter orifice 24, 26 to secure the control module 112 to the bladder wall 15. For example, barbs 144, such as spiral retention barbs, may engage with the bladder wall with a twisting maneuver in a direction shown by arrow A2 (shown in FIG. 5). The spiral barbs 144 may be removed from the bladder wall by twisting the control module 112 in an opposite direction, as shown by arrow A3. In some examples, the spiral barbs 144 can be retractable. In that case, a user may advance the pump assembly 100 into the urinary tract with the proximal end of the control module 112 in contact with the bladder wall. Once the control module 112 is in place, the user deploys the barbs 144 such that the barbs 144 embed into the bladder wall to maintain positioning of the control module 112 and/or pump module 110 within the urinary tract.

### Pump module with inlet line

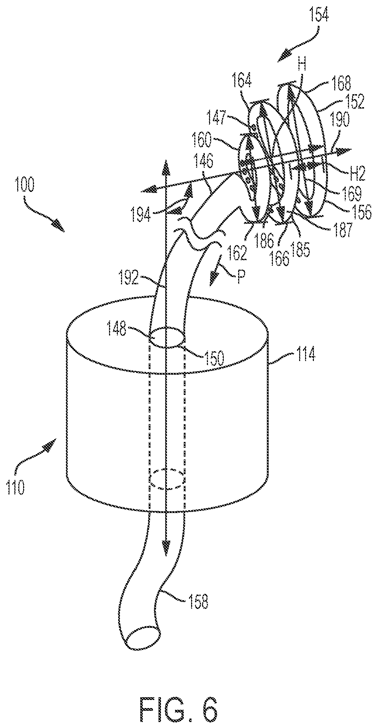

In other examples, as shown in FIG. 6, the pump module 110 can be configured to be positioned in the patient's bladder 12 rather than in the ureter 24, 26. In that case, the housing 114 may be large enough to also enclose electronic components of the control module, such as a computer processor and battery. In such a configuration, the pump assembly 100 can further comprise an inlet line 146 or drainage lumen or channel extending from the pump module 110 into the patient's ureter 8, 10 and/or renal pelvis 14, 16. For example, the inlet line 146 can be a substantially tubular conduit comprising a proximal end 148 mounted to a fluid inflow port 150 of the pump module 110 and a distal end 152 for placement in the ureter 8, 10 and/or renal pelvis 14, 16. In some examples, the inlet line 146 can have an external diameter ranging from about 0.33 mm to about 3.0 mm, or about 1.0 mm to 2.0 mm. In some examples, the internal diameter of the inlet line 146 can range from about 0.165 mm to about 2.39 mm, or from about 1.0 mm to 2 mm, or about 1.25 mm to about 1.75 mm. In one example, the inlet line 146 is 6 Fr and has an outer diameter of 2.0 ± 0.1 mm. The inlet line 146 can be formed from one or more suitable biocompatible materials, such as materials used for conventional urinary tract catheters. Exemplary materials can comprise polyvinyl chloride, polytetrafluoroethylene (PTFE) such as Teflon^{®}, silicone coated latex, and/or silicone.

In some examples, the inlet line 146 comprises a plurality of openings 147 or drainage holes extending through a sidewall thereof for drawing fluid from the ureter and/or kidney into an interior lumen or flow channel of the line 146. In other examples, portions of the inlet line 146 can be formed from a porous and/or water absorbent material, such as a sponge, mesh, woven fiber, or similar material. In that case, fluid can be drawn into the interior of the lumen or flow channel through the porous material.

In some examples, the distal end 152 of the inlet line 146 comprises a retention portion, indicated generally at 154, for maintaining the position of the inlet line 146 at a desired fluid collection position proximate to or within the ureter 8, 10 and/or renal pelvis 14, 16. Non-limiting examples of suitable retention portions are disclosed in U.S. Patent Application Publication Nos. 2017/0021128 and 2017/0021129, and PCT International Publication No. WO 2017/015345.

In some examples, the retention portion 154 is configured to be flexible and bendable to permit positioning of the retention portion 154 in the ureter and/or renal pelvis. The retention portion 154 is desirably sufficiently bendable to absorb forces exerted on the inlet line 146 and to prevent such forces from being translated to the ureters. For example, if the retention portion 154 is pulled in the proximal direction P (shown in FIG. 6) toward the patient's bladder, the retention portion 154 can be sufficiently flexible to begin to unwind or be straightened so that it can be drawn through the ureter. Similarly, when the retention portion 154 can be reinserted into the renal pelvis or other suitable region within the ureter, it can be biased to return to its deployed configuration.

In some examples, the retention portion 154 is integral with the inlet line 146. In that case, the retention portion 154 can be formed by imparting a bend or curl to the inlet line 146 that is sized and shaped to retain the retention portion at a desired fluid collection location. Suitable bends or coils can include a pigtail coil, corkscrew coil, and/or helical coil. For example, the retention portion 154 can comprise one or more radially and longitudinally extending helical coils configured to contact and passively retain the inlet line 146 within the ureter 8, 10 proximate to or within the renal pelvis 14, 16. In other examples, the retention portion 154 is formed from a radially flared or tapered portion of the inlet line 146. For example, the retention portion 154 can further comprise a fluid collecting portion, such as a tapered or funnel-shaped inner surface. In other examples, the retention portion 154 can comprise a separate element connected to and extending from the inlet line 146.

Referring now to FIG. 6, exemplary retention portions 154 comprise a plurality of helical coils, such as one or more full coils and one or more half or partial coils, are illustrated. The retention portion 154 can be capable of moving between a contracted position and the deployed position with the plurality of helical coils. For example, a substantially straight guidewire can be inserted through the retention portion 154 to maintain the retention portion 154 in a substantially straight contracted position. When the guidewire is removed, the retention portion 154 can transition to its coiled configuration. In some examples, the coils 156 extend radially and longitudinally at the distal portion 152 of the inlet line 146. In some examples, the retention portion 154 can comprise one or more coils 156, each coil having an outer coil diameter sufficient to contact at least a portion of the interior wall of the ureter and/or renal pelvis to maintain the inlet line 146 at a desired position in the patient's ureter and/or renal pelvis.

In some examples, the coiled retention portion comprises at least a first coil 160 having a first outer diameter 162; at least a second coil 164 having a second outer diameter 166, the first outer diameter 162 being less than the second outer diameter 166, the second coil 164 being closer to an end of the distal portion of the drainage channel than the first coil 160. The first outer diameter 162 can range from about 12 mm to about 16 mm, or about 13 mm to about 15 mm. The second outer diameter 166 can range from about 16 mm to about 20 mm, or about 17 mm to about 19 mm. The retention portion can further comprise a third coil 168 extending about the axis of the retention portion, the third coil 168 having an outer diameter 169 greater than or equal to either the first coil outer diameter 162 or the second coil outer diameter 166, the third coil 168 being closer to an end of the distal portion of the drainage channel than the second coil 164. The third outer diameter 169 can range from about 12 mm to about 20 mm. The coiled retention portion 154 can have a height H ranging from about 14 mm to about 18 mm.

In some examples, prior to insertion or after insertion into the patient's body, the central axis 190 of the retention portion 154 can be coextensive with, generally parallel to, curved or angled relative to the central axis 192 of the flow channel of the drainage lumen. In some examples, at least a portion of the axis 190 of the retention portion 154 extends at an angle 194 from the central axis 192 from 0 to about 90 degrees, or about 15 degrees to about 75 degrees, or about 45 degrees.

In some examples, prior to insertion into a patient's urinary tract, a portion of the drainage channel that is proximal to the retention portion defines a straight or curvilinear central axis, and wherein, when deployed, the coil(s) of the retention portion extend about the central axis 190 of the retention portion 154 that is at least partially coextensive or coextensive with the straight or curvilinear central axis 192 of the portion of the flow channel 122.

In some examples, multiple coils 156 can have the same inner and/or outer diameter D and height H. In that case, the outer diameter 162, 166, 169 of the coils 156 can range from about 10 mm to about 30 mm. The height H2 between the centerline of each coil 156 can range from about 3 mm to about 10 mm.

In some examples, the retention portion 154 is configured to be inserted in the tapered portion of the renal pelvis. For example, the outer diameter D of the coils 156 can increase toward the distal end 152 of the inlet line 146, resulting in a helical structure having a tapered or partially tapered configuration. For example, the distal or maximum outer diameter 169 of the tapered helical portion ranges from about 10 mm to about 30 mm, which corresponds to the dimensions of the renal pelvis.

In some examples, the outer diameter 162, 166, 169 and/or height H2 of the coils 156 can vary in a regular or irregular fashion. For example, the outer diameter 162, 166, 169 of coils or height H2 between coils can increase or decrease by a regular amount (e.g., about 10% to about 25% between adjacent coils 156). For example, for a retention portion 154 having three coils (as shown, for example, in Fig. 6) an outer diameter 162 of a proximal-most coil or first coil 160 can range from about 6 mm to about 18 mm, an outer diameter 166 of a middle coil or second coil 164 can range from about 8 mm to about 24 mm, and an outer diameter 169 of a distal-most or third coil 168 can range from about 10 mm to about 30 mm.

Other non-limiting examples of suitable retention portions, such as funnel-shaped structures, inflatable or balloon structures, porous and/or sponge-like structures, and expandable cage structures are disclosed in U.S. Patent Application Publication Nos. 2017/0021128 and 2017/0021129, and PCT International Publication No. WO 2017/015345. Some examples of suitable catheters, systems and methods of use are disclosed in U.S. Patent Application No. 15/687,064, entitled "Ureteral and Bladder Catheters and Methods of Inducing Negative Pressure to Increase Renal Perfusion", filed on August 25, 2017.

Optionally, the retention portion 154 can further comprise one or more perforations or drainage holes 147 configured to draw fluid into an interior of the inlet line 146, for example disposed on or through the sidewall of the inlet line 146 on or adjacent to the retention portion 154 to permit urine waste to flow from the outside of the inlet line 146 to the inside of the flow channel. Drainage holes 147 can be positioned in a spaced apart arrangement along a sidewall of the inlet line 146. In some examples, the retention portion 154 can further comprise an additional hole at a distal end 152 of the retention portion 154.

The drainage holes 147 can be located, for example, proximate the open distal end 152 of the inlet line 146. In other examples, perforated sections and/or drainage holes 147 are disposed along the sidewall 185 of the distal portion of the inlet line 146. The drainage holes 147 can be used for assisting in fluid collection. In other examples, the retention portion 154 is solely a retention structure and fluid collection and/or imparting negative pressure is provided by structures at other locations on the inlet line 146.

In some examples, the retention portion 154 of the inlet line 146 comprises a sidewall 185 comprising a radially inwardly facing side 186 and a radially outwardly facing side 187, and wherein a total surface area of perforations or holes 147 on the radially inwardly facing side 186 is greater than a total surface area of perforations or holes 147 on the radially outwardly facing side 187. In some examples, the radially outwardly facing side 187 is essentially free or free of perforations.

The configuration of the drainage holes 147 can be any configuration which permits the passage of fluid F1 therethrough, such as circular or non-circular. The position and size of the drainage holes 147 can vary depending upon the desired flow rate and configuration of the retention portion. The diameter of each of the drainage holes 147 can range from about 0.05 mm to 1.1 mm, about 0.7 mm to about 0.9 mm. The cross-sectional area of each drainage hole 147 ranges from about 0.002 mm² to about 1.0 mm², or about 0.35 mm² to about 0.65 mm². The distance between adjacent drainage holes 147, for example the linear distance between drainage holes 147 when the coils are straightened, can range from about 20 mm to about 25 mm, or about 21 mm to about 23 mm. The drainage holes 147 can be spaced in any arrangement, for example, linear or offset. The total cross-sectional area of all of the drainage holes 147 can range from about 0.002 mm² to about 10 cm², about 0.02 mm² to about 8 cm², or about 0.2 mm² to about 5 cm². In some examples, the drainage holes 147 can be non-circular, and can have a cross-section area of about 0.00002 mm² to about 0.79 mm² or about 0.02 mm² to about 0.8 mm²,.

In some examples, the drainage holes 147 are located around the entire periphery of the sidewall of the inlet line 146 to increase an amount of fluid that can be drawn into the flow channel. In other examples, the drainage holes 147 can be disposed essentially only on the radially inwardly facing side 186 of the coils 156 to prevent occlusion or blockage of the drainage holes 147, and the outwardly facing side 187 of the coils may be essentially free of drainage holes 147. For example, when negative pressure is induced in the ureter and/or renal pelvis, mucosal tissue of the ureter and/or kidney may be drawn against the retention portion 154 and may occlude some drainage holes 147 on the outer periphery of the retention portion 154. Drainage holes 147 located on the radially inward side of the retention structure would not be appreciably occluded when such tissues contact the outer periphery of the retention portion 154. Further, risk of injury to the tissues from pinching or contact with the drainage holes 147 can be reduced or ameliorated.

In some examples, the retention portion 154 can include one or more mechanical stimulation devices for providing stimulation to nerves and muscle fibers in adjacent tissues of the ureter(s) and renal pelvis. For example, the mechanical stimulation devices can include linear or annular actuators embedded in or mounted adjacent to portions of the sidewall of the catheter tube 122 and configured to emit low levels of vibration. In some examples, mechanical stimulation can be provided to portions of the ureters and/or renal pelvis to supplement or modify therapeutic effects obtained by application of negative pressure. While not intending to be bound by theory, it is believed that such stimulation affects adjacent tissues by, for example, stimulating nerves and/or actuating peristaltic muscles associated with the ureter(s) and/or renal pelvis. Stimulation of nerves and activation of muscles may produce changes in pressure gradients or pressure levels in surrounding tissues and organs which may contribute to or, in some cases, enhance therapeutic benefits of negative pressure therapy.

In some examples, the pump module 110 further comprises an outlet line 158 extending from the pump module 110 to a portion of the patient's urinary tract 2. The outlet line 158 can formed from a similar material and have similar dimensions to the inlet line 146. The outlet line 158 may extend from the bladder, through the urethral sphincter and the urethra, and to a collection container external to the body. In some examples, the length of the outlet line 158 may range from about 30 cm to about 120 cm depending on the gender and age of the patient.

### Negative pressure therapy system

Referring now to FIG. 12, the pump assembly 100 can be a component of a negative pressure therapy or treatment system 300 for providing negative pressure therapy to a patient. The system 300 comprises the pump assembly 100 in communication with one or more computer devices positioned outside of the patient's body for controlling operation of the pump assembly 100 and for receiving, processing, and analyzing data generated by indwelling components of the pump assembly 100.

**In** some examples, as shown in FIG. 12, the system 300 comprises a remote control device 310 in wired or wireless communication with the control module 112 of the pump assembly 100. The remote control device 310 can be a dedicated electronic device configured to communicate with the pump assembly 100. In other examples, the remote control device 310 is a general purpose computer device configured to execute software for communicating with and/or controlling operation of the pump assembly 100. For example, the remote control device 310 can be a handheld web-enabled computer device, such as a smart phone, tablet PC, or personal digital assistant. In other examples, the remote control device 310 can be a laptop computer, desktop computer, or computer server as is known in the art. The remote control device 310 can be located in close proximity to the patient. For example, as previously described, the remote control device 310 can be a portable device which is placed in a pocket, fanny pack, holster, or harness worn by the patient and configured to position the remote control device 310 as close to the pump assembly 100 as possible. In other examples, the remote control device 310 may be a stationary electronic device placed, for example, in a patient's house or hospital room, and configured to communicate with the pump assembly 100 by a long-range data communications protocol, such as WiFi.

In some examples, the remote control device 310 comprises a controller 312, a communications interface 314 configured to communicate with the pump assembly 100 and with other remote computer devices or networks, and optionally an electromagnetic field generator 316 configured to generate an electromagnetic field to cause the induction coil 210 to generate power.

In some examples, the remote control device 310 further comprises a feedback and/or user interface module 318 operatively connected to a feedback device, such as a visual display 320. The feedback and/or user interface module 318 is configured to receive information generated by the one or more sensors 230, 232 associated with the pump module 110 and to provide feedback to the user about operating conditions of the pump assembly 100 and/or about a physiological condition of the patient. For example, the feedback and/or user interface module 318 may be configured to cause the visual display 320 to display information about a volume and/or flow rate of urine which passes through the flow channel 122 or about an amount of negative pressure being generated by the pump module 110. In other examples, the displayed information can also include information about the pump assembly 100, such as a charge remaining of the battery 226 or estimated time until the battery 226 will need to be recharged. In some examples, information about a treatment protocol for a patient can also be displayed. For example, information about how long negative pressure will continue to be delivered to the patient or showing a pattern of positive and negative pressures to be delivered to the patient may be displayed.

In some examples, the communications interface 314 comprises a short-range data transceiver 322 configured to communicate with the communications interface of the control module 112. For example, the short-range data transceiver 322 can comprise a Bluetooth^{®} transceiver, near-field communications (e.g., RFID) transceiver, or similar data transmission device. Since the remote control device 310 is configured to be positioned as close to the pump assembly 100 as possible, the transmission range of the short-range data transceiver 322 need only be a few feet or less. In some examples, the communications interface 314 further comprises a long-range data transceiver 324 for transmitting information collected by the pump assembly 100 and remote control device 310 to a remote source such as a computer network 326, a database 328, or a web-based portal or website 330. For example, information about the patient and/or about treatment provided by the pump assembly 100 can be transmitted from the remote control device 310 to the remote database 328 for inclusion in the patient's electronic health record. A confirmation that treatment has been provided can also be transmitted to medical professionals such as a responsible physician. In some cases, the physician may be able to review the confirmation as well as physiological information about the patient using, for example, the web-based portal 330.

### Deployment

The pump assembly 100, battery 226, and/or induction coil 220 are configured to be inserted into the bladder, ureter, and/or renal pelvis through the patient's urethra. In order to facilitate placement and deployment in this manner, the pump assembly 100 described herein is configured to fit within a deployment device, such as a catheter tube and, once advanced from the tube, is configured to automatically transition to deployed positions. In some configurations, the entire assembly, including the pump module, control module, and induction coil, can be delivered into the ureter and bladder via the urethra using, for example, a 12 - 16 Fr catheter (4.0 - 5.3 mm in outer diameter). In other examples, portions of the pump assembly can be delivered through an abdominal incision or a nephrostomy or urostomy transdermal procedure.

As shown in FIGS. 13A and 13B, an exemplary deployment catheter 410 for use with the pump assembly 100 comprises a flexible elongated tube 412 comprising an open distal end 414 configured to be inserted into the urinary tract through the urethra, a proximal end 416, which can be configured to remain outside the patient's body, and a sidewall 418, such as a substantially continuous sidewall formed from a flexible medical grade plastic material, extending therebetween. For example, the elongated tube 412 can be formed from materials including biocompatible polymers, polyvinyl chloride, polytetrafluoroethylene (PTFE) such as Teflon^{®}, silicone coated latex, or silicone. In one example, the tube 412 is formed from a thermoplastic polyurethane. At least a portion or all of the catheter 410, such as the tube 412, can be coated with a hydrophilic coating to facilitate insertion and/or removal, and/or to enhance comfort. In some examples, the coating is a hydrophobic and/or lubricious coating. For example, suitable coatings can comprise ComfortCoat^{®} hydrophilic coating which is available from Koninklijke DSM N.V. or hydrophilic coatings comprising polyelectrolyte(s) such as are disclosed in United States Patent No. 8,512,795.

In some examples, the proximal end 416 of the tube 412 can comprise a hub (not shown) including a guidewire lumen port for assisting a user in positioning the catheter 410 through the urethra and into the bladder and/or ureter. The catheter 410 can be a standard deployment catheter formed from a biocompatible flexible material such as, for example, silicone rubber. The elongated tube 412 can be any standard size for insertion in the urinary tract, such as a 12 Fr to 16 Fr tube. The length of the elongated tube 412 may range from about 30 cm to about 120 cm depending on the gender and age of the patient.

As shown in FIGS. 13A and 13B, the pump assembly 100, including the pump module 110, control module 112, and induction coil 210, is configured to be positioned within the tube 412 in a contracted position. The pump assembly 100 is advanced through the tube 412 by a pusher rod 420, as shown in FIG. 13B. Once the open distal end 414 of the catheter 410 is advanced through the urinary tract to a desired position within the bladder, ureter, or kidney, a user may advance the pusher rod 420 through the elongated tube 412 to cause the components of the pump assembly 100 to exit the tube 412 through the open distal end 414 thereof. Once clear of the tube 412, structures of the pump module 110 and control module 112 may deploy from a contracted position to a deployed position. For example, radially extending barbs 140 (shown in FIG. 4) may extend radially outward from the sidewall 120 of the pump module 110 and come into contact with the interior ureter wall to maintain positioning of the pump module 110 within the ureter. In a similar manner, once the induction coil 210 extends past the open distal end 414 of the elongated tube 412, it may uncurl in the manner previously described. In some examples, the induction coil 210 may be biased to its uncurled state, in which case it may uncurl automatically as soon as it is removed from the elongated tube 412. In other examples, a user may manually cause the induction coil 210 to uncurl by actuating a release button or triggering wire.

In order to deploy the pump assembly 100 in a patient's urinary tract, a medical professional may first advance a guidewire to a desired position in the bladder and/or ureter. In some instances, visualization devices, such as a cystoscope, may be used to obtain visualization of the bladder and ureter openings to assist in positioning of the distal end of the guidewire. The delivery catheter 410 can be delivered over the guidewire. For example, a medical professional may insert the delivery catheter 410 over the guidewire and advance the distal end 414 of the catheter 410 over the guidewire toward the ureteral orifice 24, 26. Once the distal end 414 of the catheter 410 is in place, the medical professional can begin to push the pump assembly 100 from the elongated tube 412 by advancing the pusher rod 420 through the deployment elongated tube 412. As it is expelled from the elongated tube 412, the open distal end 118 of the pump module 110 is advanced through the ureteral orifice 24, 26 and into a distal end of the patient's ureter. The control module 112 and induction coil 220 can remain in the bladder.

In some examples, as previously discussed, housings 114, 128 of the pump module 110 and/or control module 112 can include retractable or permanently extending barbs 140, 144 for mounting the module(s) 110, 112 to a surface of the ureter, renal pelvis, bladder, or urethra. In some examples, once the pump assembly 100 comprising the pump module 110 and control module 112 is in position in the patient's urinary tract, the user may actuate a release mechanism to cause the barbs 140, 144 to extend toward the interior wall of the ureter, renal pelvis, bladder, or urethra r. In other examples, the barbs 140, 144 may extend automatically as the elongated tube is being retracted. Retraction of the elongated tube 412 also causes the deployable induction coil 210 to uncurl from a rolled configuration to a substantially flat configuration.

### Bladder pump assembly

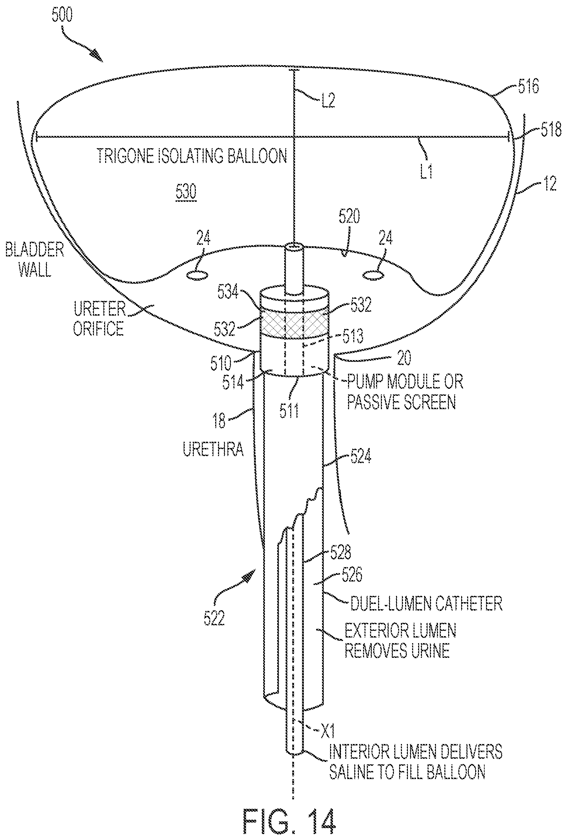

According to another example of the disclosure, a bladder pump assembly 500 for inducement of negative pressure in the bladder is illustrated in FIG. 14. Negative pressure induced in the bladder also acts on the ureter and kidneys to draw urine from the kidneys. As discussed herein, such negative pressure is believed to enhance urine production resulting in physiological benefits, such as reduced venous congestion and reduced risk of acute kidney injury.

As shown in FIG. 14, the bladder pump assembly, shown generally as 500, comprises a pump module 510. The pump module 510 can be substantially similar to previously described pump modules and can include, for example, impeller or piezoelectric configurations as shown in FIGS. 7 and 8. In some examples, the pump module 510 comprises an annular housing 512 sized for placement in the patient's bladder. For example, the pump module 510 can be placed adjacent to the urethral opening or sphincter 20, such that the sphincter 20 seals about an outer circumference 514 of the housing to prevent fluid from leaking from the bladder.

In some examples, the pump module 510 comprises fluid entry holes or ports 532 extending through a sidewall 534 of the module 510 for drawing fluid into a central channel 513 of the module 510. In some examples, a diameter of each of the drainage holes or ports 532 is about 0.5 mm to 2.0 mm or about 0.75 mm to 1.0 mm. The distance between adjacent drainage holes or ports 532 about the circumference of the sidewall 534 can be about 5 mm to about 30 mm or about 10 mm to 20 mm. The holes or ports 532 can comprise a circular opening, a square shaped opening, an elliptical opening, and any combination thereof. In some examples, the holes or ports 532 can be covered by a screen or filter for preventing solid materials from being draw into the pump module 510.

The assembly 500 further comprises a bladder wall support 516 extending from the pump module 510. The bladder wall support 516 is configured to prevent a superior portion 15a of the bladder wall 15 from collapsing when negative pressure is applied to the bladder 12, ureter 8, 10, and kidneys 4, 6 by the pump module 510. In particular, the bladder wall support 516 maintains at least the superior portion 15a of the bladder 12 in an un-collapsed state in which the ureteral orifices 24, 26 are not occluded by the collapsed bladder wall 15. Exemplary bladder wall supports, which can be used in a negative pressure therapy system for providing negative pressure to portions of the urinary tract, such as the bladder, ureters, and kidneys, are described in International Publication No. WO 2017/015345 entitled "Catheter Device and Method for Inducing Negative Pressure in a Patient's Bladder.

In some examples, the bladder wall support 516 comprises an inflatable balloon 518 configured to expand from a collapsed state to an inflated state. The balloon 518 is configured to isolate the trigone region 22 of the bladder 12 from the superior bladder wall 15a, thereby preventing the superior bladder wall 15a from collapsing into the trigone region 22 when negative pressure is applied thereto. In some examples, the balloon 518 can be about 1.0 cm to 2.3 cm in diameter, and preferably about 1.9 cm (0.75 in) in diameter. The balloon 518 is preferably formed from a flexible material including, for example, biocompatible polymers, polyvinyl chloride, polytetrafluoroethylene (PTFE) such as Teflon^{®}, silicone coated latex, or silicone.

As shown in FIG. 14, in some examples, the balloon 518 has a substantially flattened or elongated cross section having a maximum inflated width L1 of, for example, about 15 cm or less or about 10 cm or less, which is greater than its maximum inflated height L2 of, for example, about 5 cm or less or 2.5 cm or less. The width L1 generally corresponds to the width of a patient's bladder. In some examples, a bottom or proximal surface 520 of the balloon 518 is a concave surface offset from the proximal surface of the bladder by about 1 cm to about 3 cm so as to permit free flow of urine from the ureter orifice 24.

The pump assembly 500 further comprises a drainage conduit or tube 522 comprising an elongated tubular member 524. As previously described, a tubular member for insertion in the urinary system, such as tubular member 524, can be formed from any suitable flexible material including biocompatible polymers, polyvinyl chloride, polytetrafluoroethylene (PTFE) such as Teflon^{®}, silicone coated latex, or silicone. In some examples, at least a portion or all of the tubular member can be coated with a hydrophilic coating to facilitate insertion and/or removal and/or to enhance comfort. In some examples, the coating is a hydrophobic and/or lubricious coating. For example, a suitable coating can comprise ComfortCoat^{®} hydrophilic coating.

In some examples, the elongated tubular member 524 extends from a proximal end 511 of the pump module 510, through the urethra 18, and extends to the outside of the patient's body. The drainage catheter 522 may be connected to a fluid collection container, such as a urine collection bag or pouch (not shown). The drainage catheter 522 can be a single or multi-lumen catheter comprising one or more drainage lumens 526 in fluid communication with the channel 513 of the pump module 510. In some examples, the drainage catheter 522 further comprises an inflation lumen 528 in fluid communication with an interior 530 of the inflatable balloon 518. As shown in FIG. 14, in some examples, the inflation lumen 528 extends through the pump module 510 and into the interior 530 of the balloon 518. The inflation lumen 528 is used to deliver a filling material, such as saline solution, to inflate the balloon 518. When a user is prepared to remove the pump assembly 500 from the body, the balloon 518 can be deflated by emptying the filling material, such as saline solution contained in the balloon interior 520, through the inflation lumen 528 and out of the body.

In some examples, the inflation lumen 528 extends through the drainage lumen 526, as shown in FIG. 14. For example, the inflation lumen 528 may extend through the drainage lumen 526 such that a longitudinal central axis X1 of the drainage lumen 526 is substantially co-extensive with a longitudinal central axis of the inflation lumen 528. However, many different arrangements of the drainage lumen 526 and inflation lumen 528 may be used within the scope of the present disclosure. For example, a separate drainage lumen 526 and inflation lumen 528 may extend through the drainage catheter 522 in a side-by-side configuration. Other configurations of the drainage lumen 526 and inflation lumen 528 will also be apparent to those of ordinary skill in the art.

In use, the bladder pump assembly 500 comprising the pump module 510, bladder wall support 516, and elongated tubular member 524 is advanced through the urethra 18 into the bladder 12. The balloon 518 of the bladder wall support 516 is then expanded within the bladder 12 as shown in FIG. 14. Once the elongated member 524 and pump module 510 are in place, the urethral sphincter 20 can be permitted to seal or partially seal around the outer circumference 514 of the pump module 510. Once the balloon 518 and pump module 510 are positioned within the bladder 12, a user may actuate a pump element of the pump module 510 to draw urine from the bladder 12 into the pump module 510 through the fluid entry ports 532. The negative pressure generated by the pump module 510 also acts on more distal portions of the urinary tract. For example, the ureter(s) and kidney(s) can be exposed to the negative pressure to increase renal perfusion in the manner described herein. When the pump module 510 is actuated, fluid is drawn through the channel 513 of the pump module 510 due to motion of the pump element and expelled from the pump module 510 to the drainage lumen 526 of the drainage catheter 522. The collected fluid drains from the body through the drainage catheter 522 where it is collected in a fluid collection container, such as a bag or pouch, located outside the patient's body. Collected urine can be analyzed to monitor patient physiological condition and to confirm that the pump assembly 510 is operating and providing negative pressure in an expected manner. The pump module 510 can further comprise sensors 230, 232, such as are discussed above to monitor pump performance and/or physiological conditions as desired.

The preceding examples and embodiments of the invention have been described with reference to various examples. Modifications and alterations will occur to others upon reading and understanding the foregoing examples. Accordingly, the foregoing examples are not to be construed as limiting the disclosure.

## Claims

1. A pump assembly (100, 500) comprising:
(a) a pump module (110, 510), wherein at least a portion of the pump module is configured to be positioned within at least one of an interior portion of a ureter, an interior portion of a renal pelvis, or an interior portion of a bladder for providing negative pressure to at least one of the patient's ureter or kidney, the pump module comprising:
a housing (114, 512) comprising an open proximal end, an open distal end, and a sidewall extending therebetween defining a flow channel (122) for conducting fluid through at least one of the interior portion of the patient's ureter, the interior portion of the patient's renal pelvis, or the interior portion of the patient's bladder, wherein at least a portion of the housing is configured to be positioned within at least one of the interior portion of the ureter, the interior portion of the renal pelvis , or the interior portion of the bladder; and
a pump element (126) at least partially positioned within the channel configured to draw fluid through the channel between the open distal end and the open proximal end of the housing; and
(b) a control module (112) coupled to the pump module, the control module being configured to direct motion of the pump element to control flow rate of fluid passing through the channel, the control module comprising a housing configured to be positioned within at least one of a second interior portion of the patient's ureter, a second interior portion of the patient's renal pelvis, or a second interior portion of the patient's bladder.

2. The pump assembly of claim 1, wherein the control module comprises a control module housing (128), and wherein a maximum outer diameter of the pump module housing is less than a maximum outer diameter of the control module housing and wherein the pump module housing is preferably integrally formed with or connected to the control module housing.

3. The pump assembly of any one of the preceding claims, wherein the control module further comprises a control module housing having a maximum outer diameter which is configured to be larger than an interior diameter of the patient's ureter, such that the control module does not pass from the patient's bladder into the ureter, wherein the pump module housing is preferably integrally formed with or connected to the control module housing; and/or wherein the maximum outer diameter of the pump module housing is about 0.5 mm to about 5.0 mm.

4. The pump assembly of any one of the preceding claims, wherein the pump module housing comprises one or more retention members (140, 144) extending from the sidewall for releasably attaching a portion of the pump module housing to at least one of the interior portion of the ureter, the interior portion of the renal pelvis, or the interior portion of the bladder.

5. The pump assembly of claim 4, wherein the retention members are retractable to permit removal of the pump module from the ureter, renal pelvis or the bladder or wherein the retention members have a length when extended of less than about 3 mm.

6. The pump assembly of any one of the preceding claims, wherein the control module further comprises a control module housing comprising one or more retention members extending therefrom for releasably attaching the control module housing to an interior portion of the bladder of a patient, wherein the pump module housing is preferably integrally formed with or connected to the control module housing.

7. The pump assembly of any one of the preceding claims, wherein at least a portion of the pump module housing comprises a drainage channel comprising a distal portion configured to be positioned within at least one of a distal interior portion of the ureter and/or the interior portion of the renal pelvis of a patient, wherein the drainage channel is preferably formed integrally with the pump module housing or as a separate tube or conduit in fluid connection with the open distal end of the pump module housing.

8. The pump assembly of claim 7, wherein the distal portion of the drainage channel comprises a coil (156, 160 164) and/or wherein the coil comprises one or more perforations in a sidewall of the coil and/or wherein the coil comprises one or more perforations in an inwardly facing side of a sidewall of the coil.

9. The pump assembly of any one of the preceding claims, wherein the control module comprises a control module housing comprising a generally cylindrical housing comprising an open distal end connected to the open proximal end of the pump module housing, an open proximal end, and a flow channel in fluid communication with the flow channel of the pump module housing and extending between the proximal end and the distal end of the control module housing.

10. The pump assembly of any one of the preceding claims, wherein the control module comprises a control module housing which is separate from the pump module housing and wherein electronic circuitry of the control module is operatively connected to the pump module via a wired or wireless connection.

11. The pump assembly of any one of the preceding claims, wherein the pump element comprises an impeller (170) positioned within the flow channel of the pump module housing which rotates to draw fluid through the channel.

12. The pump assembly of any one of the preceding claims, wherein the pump element comprises a piezoelectric diaphragm (180) positioned within the channel that can be configured to alternately extend from and retract to an inner surface of the sidewall to draw fluid through the flow channel, wherein the pump module preferably further comprises a distal valve (182) positioned in a portion of the flow channel distal to the pump element and a proximal valve (184) positioned in a portion of the flow channel proximal to the pump element, wherein the distal valve and the proximal valve preferably each comprise a one-way check valve configured to produce one-directional flow of fluid through the flow channel from the distal end to the proximal end thereof.

13. The pump assembly of any one of the preceding claims, wherein the control module comprises a wireless transceiver configured to receive operating instructions from a remote device and to provide information about negative pressure treatment from the control module to the remote device.

14. The pump assembly of any one of the preceding claims, further comprising an induction coil electronically coupled to at least one of the pump module or the control module for providing power thereto, the induction coil being configured to generate power when exposed to an electromagnetic field generated by a remote device positioned outside or within the patient's body, wherein the induction coil preferably comprises a conductive wire at least partially disposed on a flexible substrate wherein the flexible substrate is preferably transitionable from a rolled configuration in which the flexible substrate is rolled about a central axis thereof to a size suitable for delivery through a catheter to a deployed configuration in which the flexible substrate is at least partially unrolled from the rolled configuration.

15. A system for providing negative pressure therapy to a patient's ureter and/or kidney, the system comprising:
a pump assembly according to any of the previous claims; and
a remote control device (310) in wired or wireless communication with the control module, the remote control device being configured to provide instructions to the control module for operating the pump assembly and to receive information from the control module about at least one of the pump module or the patient.

16. The system of claim 15, further comprising a remote database comprising electronic patient health records, and wherein the remote control device is configured to wirelessly transmit information about the patient to the remote database.

17. The system of claim 15 or 16, further comprising sensors in fluid communication with the flow channel of the pump module housing, the sensors being configured to measure a pump operating parameter or a physiological condition of the patient based on sensed information about fluid passing through the flow channel.

18. The system of any of claims 15 to 17 wherein the remote control device further comprises a display (320), and wherein the remote control device is configured to display the information received from the control module about at least one of the pump module or the patient on the display.

## Patentansprüche

1. Eine Pumpenanordnung (100, 500), umfassend:
(a) ein Pumpenmodul (110, 510), wobei zumindest ein Teil des Pumpenmoduls so konfiguriert ist, dass er innerhalb zumindest eines der folgenden Bereiche positioniert werden kann: eines inneren Abschnitts eines Ureters, eines inneren Abschnitts eines Nierenbeckens oder eines inneren Abschnitts einer Blase, um einen Unterdruck an zumindest einem der Ureter oder einer Niere des Patienten zu erzeugen, wobei das Pumpenmodul umfasst:
ein Gehäuse (114, 512) mit einem offenen proximalen Ende, einem offenen distalen Ende und einer dazwischen verlaufenden Seitenwand, die einen Strömungskanal (122) zur Leitung von Flüssigkeit durch mindestens einen der folgenden Abschnitte definiert: den inneren Abschnitt des Ureters des Patienten, den inneren Abschnitt des Nierenbeckens des Patienten oder den inneren Abschnitt der Blase des Patienten, wobei zumindest ein Abschnitt des Gehäuses so konfiguriert ist, dass er innerhalb zumindest eines der inneren Abschnitte des Ureters, des Nierenbeckens oder der Blase positioniert werden kann; und
ein Pumpenelement (126), das zumindest teilweise innerhalb des Kanals angeordnet ist und so ausgebildet ist, dass es Flüssigkeit durch den Kanal zwischen dem offenen distalen Ende und dem offenen proximalen Ende des Gehäuses ansaugt; und
(b) ein mit dem Pumpenmodul gekoppeltes Steuermodul (112), wobei das Steuermodul so konfiguriert ist, dass es die Bewegung des Pumpenelements steuert, um die Strömungsrate der durch den Kanal strömenden Flüssigkeit zu regeln, wobei das Steuermodul ein Gehäuse umfasst, das so konfiguriert ist, dass es in mindestens einem der folgenden Teile positioniert werden kann: einem zweiten inneren Abschnitt des Ureters des Patienten, einem zweiten inneren Abschnitt des Nierenbeckens des Patienten oder einem zweiten inneren Abschnitt der Blase des Patienten.

2. Die Pumpenanordnung nach Anspruch 1, wobei das Steuermodul ein Steuermodulgehäuse (128) umfasst, und wobei ein maximaler Außendurchmesser des Pumpenmodulgehäuses kleiner ist als ein maximaler Außendurchmesser des Steuermodulgehäuses und wobei das Pumpenmodulgehäuse vorzugsweise einstückig mit dem Steuermodulgehäuse ausgebildet oder mit diesem verbunden ist.

3. Die Pumpenanordnung nach einem der vorstehenden Ansprüche, wobei das Steuermodul ferner ein Steuermodulgehäuse mit einem maximalen Außendurchmesser umfasst, der so konfiguriert ist, dass er größer ist als ein Innendurchmesser des Ureters des Patienten, sodass das Steuermodul nicht von der Blase des Patienten in den Ureter gelangt, wobei das Pumpenmodulgehäuse vorzugsweise einstückig mit dem Steuermodulgehäuse ausgebildet oder mit diesem verbunden ist; und/oder wobei der maximale Außendurchmesser des Pumpenmodulgehäuses etwa 0,5 mm bis etwa 5,0 mm beträgt.

4. Die Pumpenanordnung nach einem der vorstehenden Ansprüche, wobei das Pumpenmodulgehäuse ein oder mehrere Halteelemente (140, 144) umfasst, die sich von der Seitenwand erstrecken, um einen Abschnitt des Pumpenmodulgehäuses lösbar an mindestens einem der folgenden Bereiche zu befestigen: dem inneren Abschnitt des Ureters, dem inneren Abschnitt des Nierenbeckens oder dem inneren Abschnitt der Blase.

5. Die Pumpenanordnung nach Anspruch 4, wobei die Halteelemente rückziehbar sind, um die Entfernung des Pumpenmoduls aus dem Ureter, dem Nierenbecken oder der Blase zu ermöglichen, oder wobei die Halteelemente im ausgefahrenen Zustand eine Länge von weniger als etwa 3 mm aufweisen.

6. Die Pumpenanordnung nach einem der vorstehenden Ansprüche, wobei das Steuermodul ferner ein Steuermodulgehäuse umfasst, das ein oder mehrere sich davon erstreckende Halteelemente aufweist, um das Steuermodulgehäuse lösbar an einem inneren Abschnitt der Blase eines Patienten zu befestigen, wobei das Pumpenmodulgehäuse vorzugsweise einstückig mit dem Steuermodulgehäuse ausgebildet oder mit diesem verbunden ist.

7. Die Pumpenanordnung nach einem der vorstehenden Ansprüche, wobei zumindest ein Abschnitt des Pumpenmodulgehäuses einen Drainagekanal umfasst, der einen distalen Abschnitt aufweist, der so konfiguriert ist, dass er innerhalb zumindest eines von einem distalen inneren Abschnitt des Ureters und/oder dem inneren Abschnitt des Nierenbeckens eines Patienten positioniert werden kann, wobei der Drainagekanal vorzugsweise einstückig mit dem Pumpenmodulgehäuse ausgebildet ist oder als separates Rohr oder separate Leitung in Fluidverbindung mit dem offenen distalen Ende des Pumpenmodulgehäuses vorliegt.

8. Die Pumpenanordnung nach Anspruch 7, wobei der distale Abschnitt des Drainagekanals eine Windung (156, 160, 164) umfasst und/oder wobei die Windung eine oder mehrere Perforationen in einer Seitenwand der Windung umfasst und/oder wobei die Windung eine oder mehrere Perforationen in einer nach innen gerichteten Seite einer Seitenwand der Windung umfasst.

9. Die Pumpenanordnung nach einem der vorstehenden Ansprüche, wobei das Steuermodul ein Steuermodulgehäuse umfasst, das ein im Wesentlichen zylindrisches Gehäuse umfasst, das ein offenes distales Ende, das mit dem offenen proximalen Ende des Pumpenmodulgehäuses verbunden ist, ein offenes proximales Ende und einen Strömungskanal aufweist, der in Fluidverbindung mit dem Strömungskanal des Pumpenmodulgehäuses steht und sich zwischen dem proximalen Ende und dem distalen Ende des Steuermodulgehäuses erstreckt.

10. Die Pumpenanordnung nach einem der vorstehenden Ansprüche, wobei das Steuermodul ein Steuermodulgehäuse umfasst, das vom Pumpenmodulgehäuse getrennt ist, und wobei eine elektronische Schaltung des Steuermoduls über eine drahtgebundene oder drahtlose Verbindung mit dem Pumpenmodul wirkverbunden ist.

11. Die Pumpenanordnung nach einem der vorstehenden Ansprüche, wobei das Pumpenelement ein Laufrad (170) umfasst, das innerhalb des Strömungskanals des Pumpenmodulgehäuses angeordnet ist und sich dreht, um Flüssigkeit durch den Kanal zu saugen.

12. Die Pumpenanordnung nach einem der vorstehenden Ansprüche, wobei das Pumpenelement eine piezoelektrische Membran (180) umfasst, die innerhalb des Kanals angeordnet ist und so konfiguriert werden kann, dass sie sich abwechselnd von einer Innenfläche der Seitenwand weg ausdehnt und zu dieser zurückzieht, um Flüssigkeit durch den Strömungskanal zu saugen, wobei das Pumpenmodul vorzugsweise ferner ein distales Ventil (182), das in einem dem Pumpenelement distalen Abschnitt des Strömungskanals angeordnet ist, sowie ein proximales Ventil (184), das in einem dem Pumpenelement proximalen Abschnitt des Strömungskanals angeordnet ist, umfasst, wobei das distale Ventil und das proximale Ventil vorzugsweise jeweils ein Einweg-Rückschlagventil umfassen, das so konfiguriert ist, dass es einen unidirektionalen Fluidstrom durch den Strömungskanal von dessen distalem Ende zu dessen proximalem Ende erzeugt.

13. Die Pumpenanordnung nach einem der vorstehenden Ansprüche, wobei das Steuermodul einen drahtlosen Transceiver umfasst, der so konfiguriert ist, dass er Betriebsanweisungen von einer entfernten Vorrichtung empfängt und Informationen über die Unterdruck-Behandlung vom Steuermodul an die entfernte Vorrichtung übermittelt.

14. Die Pumpenanordnung nach einem der vorstehenden Ansprüche, ferner umfassend eine Induktionsspule, die elektronisch mit mindestens einem der Elemente - dem Pumpenmodul oder dem Steuermodul - gekoppelt ist, um diese mit Energie zu versorgen, wobei die Induktionsspule so konfiguriert ist, dass sie Energie erzeugt, wenn sie einem elektromagnetischen Feld ausgesetzt ist, das von einer ausserhalb oder innerhalb des Patientenkörpers positionierten entfernten Vorrichtung erzeugt wird, wobei die Induktionsspule vorzugsweise einen leitfähigen Draht umfasst, der zumindest teilweise auf einem flexiblen Substrat angeordnet ist, wobei das flexible Substrat vorzugsweise von einer aufgerollten Konfiguration, in der das flexible Substrat um eine Hauptachse desselben auf eine für die Zuführung durch einen Katheter geeignete Größe in eine eingesetzte Konfiguration übergehen kann, in der das flexible Substrat zumindest teilweise aus der aufgerollten Konfiguration abgewickelt ist.

15. Ein System zur Durchführung einer Unterdrucktherapie am Ureter und/oder an der Niere eines Patienten, das System umfassend:
eine Pumpenanordnung gemäß einem der vorhergehenden Ansprüche; und
ein Fernsteuerungsgerät (310), das über eine drahtgebundene oder drahtlose Verbindung mit dem Steuermodul kommuniziert, wobei das Fernsteuerungsgerät so konfiguriert ist, dass es dem Steuermodul Befehle zum Betrieb der Pumpenanordnung übermittelt und vom Steuermodul Informationen über mindestens eines der folgenden Elemente empfängt: das Pumpenmodul oder den Patienten.

16. Das System nach Anspruch 15, das ferner eine entfernte Datenbank umfasst, die elektronische Patientenakten enthält, und wobei das Fernsteuerungsgerät so konfiguriert ist, dass sie Informationen über den Patienten drahtlos an die entfernte Datenbank überträgt.

17. Das System nach Anspruch 15 oder 16, das ferner Sensoren umfasst, die in Fluidverbindung mit dem Strömungskanal des Pumpenmodulgehäuses stehen, wobei die Sensoren so konfiguriert sind, dass sie einen Pumpenbetriebsparameter oder einen physiologischen Zustand des Patienten auf der Grundlage von erfassten Informationen über die durch den Strömungskanal strömende Flüssigkeit messen.

18. Das System nach einem der Ansprüche 15 bis 17, wobei das Fernsteuerungsgerät ferner eine Anzeige (320) umfasst und wobei das Fernsteuerungsgerät so konfiguriert ist, dass sie die vom Steuermodul empfangenen Informationen über mindestens eines der Elemente - das Pumpenmodul oder den Patienten - auf der Anzeige anzeigt.

## Revendications

1. Ensemble de pompe (100, 500) comprenant :
(a) un module de pompe (110, 510), dans lequel au moins une partie du module de pompe est configurée pour être positionnée à l'intérieur d'au moins l'une des parties suivantes : une partie interne d'un uretère, une partie interne d'un bassinet rénal ou une partie interne d'une vessie, afin de fournir une pression négative à au moins l'un des uretères ou des reins du patient, le module de pompe comprenant :
un boîtier (114, 512) comprenant une extrémité proximale ouverte, une extrémité distale ouverte et une paroi latérale s'étendant entre celles-ci, définissant un canal d'écoulement (122) destiné à acheminer un fluide à travers au moins l'une des parties internes de l'uretère du patient, la partie interne du bassinet rénal du patient, ou la partie interne de la vessie du patient, dans lequel au moins une partie du boîtier est configurée pour être positionnée à l'intérieur d'au moins l'une parmi la partie interne de l'uretère, la partie interne du bassinet rénal ou la partie interne de la vessie ; et
un élément de pompage (126) positionné au moins partiellement à l'intérieur du canal, configuré pour aspirer du fluide à travers le canal entre l'extrémité distale ouverte et l'extrémité proximale ouverte du boîtier ; et
(b) un module de commande (112) couplé au module de pompage, le module de commande étant configuré pour diriger le mouvement de l'élément de pompage afin de contrôler le débit du fluide passant à travers le canal, le module de commande comprenant un boîtier configuré pour être positionné à l'intérieur d'au moins l'une parmi une deuxième partie interne de l'uretère du patient, une deuxième partie interne du bassinet rénal du patient ou une deuxième partie interne de la vessie du patient.

2. Ensemble de pompe selon la revendication 1, dans lequel le module de commande comprend un boîtier de module de commande (128), et dans lequel le diamètre extérieur maximal du boîtier du module de pompe est inférieur au diamètre extérieur maximal du boîtier du module de commande, et dans lequel le boîtier du module de pompe est de préférence formé d'un seul tenant avec le boîtier du module de commande ou relié à celui-ci.

3. Ensemble de pompe selon l'une quelconque des revendications précédentes, dans lequel le module de commande comprend en outre un boîtier de module de commande ayant un diamètre extérieur maximal qui est configuré pour être supérieur au diamètre intérieur de l'uretère du patient, de sorte que le module de commande ne passe pas de la vessie du patient dans l'uretère, dans lequel le boîtier du module de pompe est de préférence formé d'un seul tenant avec le boîtier du module de commande ou relié à celui-ci ; et/ou dans lequel le diamètre extérieur maximal du boîtier du module de pompe est compris entre environ 0,5 mm et environ 5,0 mm.

4. Ensemble de pompe selon l'une quelconque des revendications précédentes, dans lequel le boîtier du module de pompe comprend un ou plusieurs éléments de retenue (140, 144) s'étendant depuis la paroi latérale pour fixer de manière amovible une partie du boîtier du module de pompe à au moins l'une parmi la partie interne de l'uretère, la partie interne du bassinet rénal ou la partie interne de la vessie.

5. Ensemble de pompe selon la revendication 4, dans lequel les éléments de retenue sont rétractables pour permettre le retrait du module de pompe de l'uretère, du bassinet rénal ou de la vessie, ou dans lequel les éléments de retenue ont, lorsqu'ils sont étendus, une longueur inférieure à environ 3 mm.

6. Ensemble de pompe selon l'une quelconque des revendications précédentes, dans lequel le module de commande comprend en outre un boîtier de module de commande comprenant un ou plusieurs éléments de retenue s'étendant à partir de celui-ci pour fixer de manière amovible le boîtier du module de commande à une partie interne de la vessie d'un patient, le boîtier du module de pompe étant de préférence formé d'un seul tenant avec le boîtier du module de commande ou relié à celui-ci.

7. Ensemble de pompe selon l'une quelconque des revendications précédentes, dans lequel au moins une partie du boîtier du module de pompe comprend un canal de drainage comportant une partie distale configurée pour être positionnée à l'intérieur d'au moins l'une parmi une partie intérieure distale de l'uretère et/ou la partie intérieure du bassinet rénal d'un patient, le canal de drainage étant de préférence formé d'un seul tenant avec le boîtier du module de pompe ou sous la forme d'un tube ou d'un conduit séparé en communication fluidique avec l'extrémité distale ouverte du boîtier du module de pompe.

8. Ensemble de pompe selon la revendication 7, dans lequel la partie distale du canal de drainage comprend une spirale (156, 160, 164) et/ou dans lequel la spirale comprend une ou plusieurs perforations dans une paroi latérale de la spirale et/ou dans lequel la spirale comprend une ou plusieurs perforations dans une face tournée vers l'intérieur d'une paroi latérale de la spirale.

9. Ensemble de pompe selon l'une quelconque des revendications précédentes, dans lequel le module de commande comprend un boîtier de module de commande comprenant un boîtier généralement cylindrique comportant une extrémité distale ouverte reliée à l'extrémité proximale ouverte du boîtier du module de pompe, une extrémité proximale ouverte, et un canal d'écoulement en communication fluidique avec le canal d'écoulement du boîtier du module de pompe et s'étendant entre l'extrémité proximale et l'extrémité distale du boîtier du module de commande.

10. Ensemble de pompe selon l'une quelconque des revendications précédentes, dans lequel le module de commande comprend un boîtier de module de commande qui est distinct du boîtier du module de pompe et dans lequel les circuits électroniques du module de commande sont reliés de manière opérationnelle au module de pompe via une connexion filaire ou sans fil.

11. Ensemble de pompe selon l'une quelconque des revendications précédentes, dans lequel l'élément de pompage comprend une roue à aubes (170) positionnée à l'intérieur du canal d'écoulement du boîtier du module de pompe, qui tourne pour aspirer du fluide à travers le canal.

12. Ensemble de pompe selon l'une quelconque des revendications précédentes, dans lequel l'élément de pompage comprend un diaphragme piézoélectrique (180) positionné à l'intérieur du canal, qui peut être configuré pour s'écarter alternativement d'une surface interne de la paroi latérale et se rétracter vers celle-ci de la paroi latérale afin d'aspirer du fluide à travers le canal d'écoulement, dans lequel le module de pompe comprend de préférence en outre une valve distale (182) positionnée dans une partie du canal d'écoulement distale par rapport à l'élément de pompage et une valve proximale (184) positionnée dans une partie du canal d'écoulement proximale par rapport à l'élément de pompage, dans lequel la valve distale et la valve proximale comprennent de préférence chacune un clapet anti-retour unidirectionnel configuré pour produire un écoulement unidirectionnel de fluide à travers le canal d'écoulement, de son extrémité distale vers son extrémité proximale.

13. Ensemble de pompe selon l'une quelconque des revendications précédentes, dans lequel le module de commande comprend un émetteur-récepteur sans fil configuré pour recevoir des instructions de fonctionnement provenant d'un dispositif distant et pour transmettre des informations concernant le traitement par pression négative du module de commande vers le dispositif distant.

14. Ensemble de pompe selon l'une quelconque des revendications précédentes, comprenant en outre une bobine d'induction couplée électroniquement au module de pompe ou au module de commande, ou aux deux, pour leur fournir de l'énergie, la bobine d'induction étant configurée pour générer de l'énergie lorsqu'elle est exposée à un champ électromagnétique généré par un dispositif distant positionné à l'extérieur ou à l'intérieur du corps du patient, dans lequel la bobine d'induction comprend de préférence un fil conducteur disposé au moins partiellement sur un substrat flexible, le substrat flexible pouvant de préférence passer d'une configuration enroulée, dans laquelle il est enroulé autour de son axe central, à une taille adaptée à l'acheminement par un cathéter, puis à une configuration déployée, dans laquelle il est au moins partiellement déroulé par rapport à la configuration enroulée.

15. Système destiné à administrer une thérapie par pression négative à l'uretère et/ou au rein d'un patient, le système comprenant :
un ensemble de pompe selon l'une quelconque des revendications précédentes ; et
un dispositif de commande à distance (310) en communication filaire ou sans fil avec le module de commande, le dispositif de commande à distance étant configuré pour fournir des instructions au module de commande afin de faire fonctionner l'ensemble de pompe et pour recevoir des informations provenant du module de commande concernant au moins l'un parmi le module de pompe et le patient.

16. Système selon la revendication 15, comprenant en outre une base de données distante contenant des dossiers médicaux électroniques du patient, et dans lequel le dispositif de commande à distance est configuré pour transmettre sans fil des informations concernant le patient à la base de données distante.

17. Système selon la revendication 15 ou 16, comprenant en outre des capteurs en communication fluidique avec le canal d'écoulement du boîtier du module de pompe, les capteurs étant configurés pour mesurer un paramètre de fonctionnement de la pompe ou un état physiologique du patient sur la base d'informations détectées concernant le fluide passant à travers le canal d'écoulement.

18. Système selon l'une quelconque des revendications 15 à 17, dans lequel le dispositif de commande à distance comprend en outre un écran (320), et dans lequel le dispositif de commande à distance est configuré pour afficher sur l'écran les informations reçues du module de commande concernant au moins l'un parmi le module de pompe et le patient.
